(19) Europäisches Patentamt
European Patent Office
Office européen des brevets

(11) Veröffentlichungsnummer : **0 120 814**
**B1**

(12) **EUROPÄISCHE PATENTSCHRIFT**

(45) Veröffentlichungstag der Patentschrift : **27.07.88**

(21) Anmeldenummer : **84810135.8**

(22) Anmeldetag : **22.03.84**

(51) Int. Cl.⁴ : **A 01 N 47/36**, C 07 D251/16, C 07 D251/46, C 07 D239/48, C 07 D239/46, C 07 D239/52, C 07 C143/78, C 07 C143/828, C 07 C161/04, C 07 C143/83, C 07 C143/70

(54) **N-Phenylsulfonyl-N'-pyrimidinyl- und -triazinylharnstoffe.**

(30) Priorität : **28.03.83 CH 1677/83**
**11.08.83 CH 4393/83**

(43) Veröffentlichungstag der Anmeldung : **03.10.84 Patentblatt 84/40**

(45) Bekanntmachung des Hinweises auf die Patenterteilung : **27.07.88 Patentblatt 88/30**

(84) Benannte Vertragsstaaten :
**AT BE CH DE FR GB IT LI NL**

(56) Entgegenhaltungen :
**EP-A- 0 044 209**
**EP-A- 0 072 347**
**EP-A- 0 106 512**
**US-A- 4 127 405**
**US-A- 4 169 719**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Meyer, Willy**
**Talweg 49**
**CH-4125 Riehen (CH)**
Erfinder : **Oertle, Konrad, Dr.**
**Vogesenstrasse 10**
**CH-4106 Therwil (CH)**

**EP 0 120 814 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Die erfindungsgemässen N-Phenylsulfonyl-N'-pyrimidinyl- und triazinylharnstoffe entsprechen der allgemeinen Formel

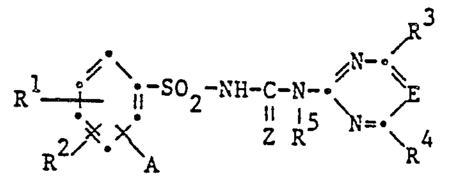

(I)

worin

$A_1$ $C_1$-$C_6$-Halogenalkyl,

$R^1$ Wasserstoff, Halogen, Nitro, Cyan, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, —CO—$R^6$, —$NR^7R^8$, —CO—$NR^9R^{10}$ oder —$SO_2$—$NR^{11}R^{12}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ und $R^4$ unabhängig voneinander Wasserstoff, Halogen, $C_1$-$C_4$-Alkyl, $C_1$-$C_4$-Halogenalkyl, $C_2$-$C_4$-Halogenalkoxy, $C_1$-$C_4$-Alkylthio, $C_1$-$C_4$-Halogenalkylthio, $C_2$-$C_4$-Alkoxyalkyl, Methoxy, Aethoxy oder —$NR^{12}R^{13}$,

$R^5$ Wasserstoff, $C_1$-$C_4$-Alkyl oder $C_1$-$C_4$-Alkoxy,

$R^6$ $C_1$-$C_4$-Alkoxy, $C_1$-$C_4$-Halogenalkoxy, $C_1$-$C_4$-Alkylthio, $C_2$-$C_6$-Alkoxyalkoxy, Wasserstoff, $C_1$-$C_4$-Alkyl oder $C_1$-$C_4$-Halogenalkyl,

$R^7$, $R^8$, $R^9$, $R^{10}$, $R^{11}$, $R^{12}$ und $R^{13}$ unabhängig voneinander Wasserstoff oder $C_1$-$C_4$-Alkyl,

E Stickstoff oder die Methinbrücke und

Z Sauerstoff oder Schwefel bedeuten,

sowie den Salzen dieser Verbindungen, mit der Massgabe, dass A nicht für Trifluormethyl, 2-Fluoräthyl, 2-Chloräthyl, 2-Bromäthyl, 2-Chlor-2-fluoräthyl, 2,2-Dichloräthyl, 2-Brom-2-chloräthyl, 2-Brom-2-fluoräthyl, 2,2-Dibromäthyl, 2-Fluorpropyl, 2-Chlorpropyl, 2-Brompropyl oder eine Gruppe —$CR^aR^bR^c$ steht, wobei $R^a$ Wasserstoff, Chlor oder $C_1$-$C_4$-Alkyl, $R^b$ Wasserstoff oder Methyl und $R^c$ Chlor oder Brom bedeuten.

Harnstoffverbindungen, Triazinverbindungen und Pyrimidinverbindungen mit herbizider Wirkung sind allgemein bekannt. Kürzlich wurden Sulfonylharnstoffverbindungen mit herbizider und pflanzenwuchsregulierender Wirkung, beispielsweise in den europäischen Patentpublikationen Nr. 44209, 44210, 44807, 44808, 44809, 72347 und 106512 beschrieben.

In den Definitionen ist unter Alkyl geradkettiges oder verzweigtes Alkyl zu verstehen, z. B. : Methyl, Aethyl, n-Propyl, i-Propyl, die vier isomeren Butyl, n-Amyl, i-Amyl, 2-Amyl, 3-Amyl, n-Hexyl oder i-Hexyl. Bevorzugt sind Methyl und Aethyl.

Unter Alkoxy ist zu verstehen : Methoxy, Aethoxy, n-Propoxy, i-Propoxy und die vier isomeren Butoxyreste, insbesondere aber Methoxy, Aethoxy oder i-Propoxy.

Beispiele für Alkylthio sind Methylthio, Aethylthio, n-Propylthio, i-Propylthio und n-Butylthio, insbesondere aber Methylthio und Aethylthio.

Beispiele für Alkylsulfinyl sind Methylsulfinyl, Aethylsulfinyl, n-Propylsulfinyl und n-Butylsulfinyl, insbesondere aber Methylsulfinyl und Aethylsulfinyl.

Beispiele für Alkylsulfonyl sind Methylsulfonyl, Aethylsulfonyl, n-Propylsulfonyl und n-Butylsulfonyl, insbesondere aber Methylsulfonyl und Aethylsulfonyl.

Unter Halogen in den Definitionen sowie in Halogenalkyl, -alkoxy, -alkylsulfinyl, -alkylsulfonyl und -alkylthio sind Fluor, Chlor und Brom, vorsugsweise jedoch Fluor und Chlor zu verstehen.

Entsprechend sind unter Halogenalkyl bzw. Halogenalkylteilen von oben definierten Substituenten $R^1$ bis $R^{13}$ beispielsweise zu verstehen : Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifulor-1,1-dichloräthyl, Pentachloräthyl, 3,3,3-Trifluorpropyl, 2,3-Dichlorpropyl, 1,1,2,3,3,3-Hexafluorpropyl, insbesondere aber Fluormethyl, Chlormethyl, Difluormethyl und Trifluormethyl.

Unter die Definition des Substituenten A fallen beispielsweise die folgenden Halogenalkylreste : Fluormethyl, Difluormethyl, Trichlormethyl, Dibrommethyl, Chlorfluormethyl, Bromfluormethyl, 1,2-Difluoräthyl, 1,2-Dibromäthyl, 1,2-Dichlorpropyl, 1,2-Dichlor-3,3,3-trifluorpropyl, 1,2-Dibrompropyl, 1,2-Dibrom-3,3,3-trifluorpropyl, 3,3,3-Trifluorpropyl, 3,3,4,4,5,5,5-Heptafluorpentyl, 4,4,4-Trifluor-2-trifluormethyl-butyl, 3,3-Difluorbutyl, 3-Chlorpropyl, 3-Fluorpropyl, 1,2-Dichloräthyl, Pentafluoräthyl, 1,1,2,2-Tetrafluoräthyl, 1,2-Dibrom-1,2-dichlorpropryl, 1,2-Dibrom-3-fluorpropyl, 1,2-Dichlor-3-fluorpropyl, 1,2,3-Trifluorpropyl, 1-Chlor-3,3,3-trifluorpropyl, 3,3-Difluorpropyl, 2,2-Difluoräthyl oder Perfluorpropyl. Vorzugsweise enthalten die Substituenten A mindestens ein Fluoratom wie z. B. 3,3,3-Trifluorpropyl, Fluormethyl, Difluormethyl, 3,3,4,4,5,5,5-Heptafluorpentyl, 4,4,4-Trifluor-2-trifluormethylbutyl, Chlorfluormethyl, Bromfluormethyl, 3,3-Difluorbutyl oder 3-Fluorpropyl, insbesondere aber 3,3,4,4,5,5,5-Heptafluorpentyl, 4,4,4-Trifluor-2-trifluormethyl-butyl, 3,3-Difluorbutyl oder 3,3,3-Trifluorpropyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorsuheben, insbesondere aber die von Natrium oder Kalium.

Beispiele für zur Salzbildung geeignete Amine sind primäre, sekundäre und tertiäre aliphatische und aromatische Amine wie Methylamin, Aethylamin, Propylamin, i-Propylamin, die vier isomeren Butylamine, Dimethylamin, Diäthylamin, Diäthanolamin, Dipropylamin, Diisopropylamin. Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Aethyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemässen Verbindungen der Formel sind die Verbindungen bevorzugt, in denen entweder

a) $R^1$ Wasserstoff ist oder
b) $R^2$ Wasserstoff ist oder
c) Z Sauerstoff ist oder
d) $R^5$ Wasserstoff ist oder
e) $R^3$ und $R^4$ zusammen höchstens 4 Kohlenstoffatome enthalten oder
f) der Rest A mindestens ein Fluoratom enthält oder
g) der Rest A die 2-Stellung am Phenylkern besetzt.

Weitere bevorzugte Gruppen von Wirkstoffen sind dadurch gekennzeichnet, dass in den Verbindungen der Formel I $R^1$, $R^2$ und $R^5$ für Wasserstoff und Z für Sauerstoff stehen oder dass $R^3$ und $R^4$ zusammen höchstens 4 Kohlenstoffatome enthalten und der Rest A die 2-Stellung des Phenylrings besetzt und mindestens ein Fluoratom enthält.

Eine besonders hervorzuhebende Gruppe von Wirkstoffen enthält Verbindungen der Formel I, in denen $R^1$, $R^2$ und $R^5$ für Wasserstoff und Z für Sauerstoff stehen, $R^3$ und $R^4$ zusammen höchstens 4 Kohlenstoffatome enthalten und der Rest A die 2-Stellung des Phenylrings besetzt und mindestens ein Fluoratom enthält.

Innerhalb der letztgenannten Gruppe von Verbindungen sind wiederum solche Verbindungen bevorzugt, in denen A für einen Rest aus der Gruppe 3,3,3-Trifulorpropyl, Fluormethyl, Difluormethyl, 3,3,4,4,5,5,5-Heptafluorpentyl, 4,4,4-Trifluor-2-trifluormethyl-butyl, Chlorfluormethyl, Bromfluormethyl, 3,3-Difluorbutyl oder 3-Fluorpropyl steht.

Als bevorzugte Einzelverbindungen sind zu nennen :

N-[2-(3,3,3-Trifluorpropyl)-phenylsulfonyl]-N'-(4,6-dimethoxy-pyrimidin-2-yl)-harnstoff,
N-[2-(3,3,3-Trifluorpropyl)-phenylsulfonyl]-N'-(4,6-dimethoxy-1,3,5-triazin-2-yl)-harnstoff,
N-[2-(3,3-Difluorbutyl)-phenylsulfonyl]-N'-(4-methoxy-6-methyl-1,3,5-triazin-2-yl)-harnstoff und
N-[2-(3,3,3-Trifluorpropyl)-phenylsulfonyl]-N'-(4-methyl-1,3,5-triazin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

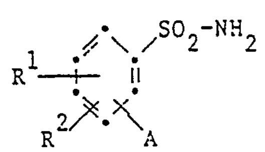

(II)

worin A, $R^1$ und $R^2$ die unter Formel I gegebene Bedeutung haben, in Gegenwart einer Base mit einem N-Pyrimidinyl- oder -Triazinylcarbamat der Formel III

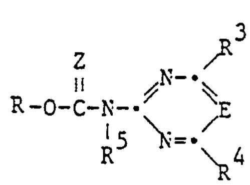

(III)

worin E, $R^3$, $R^4$, $R^5$ und Z die unter Formel I gegebene Bedeutung haben und R für Phenyl, Alkyl oder substituiertes Phenyl steht, umsetzt.

Nach einem zweiten Verfahren gelangt man zu Verbindungen der Formel I, indem man ein Phenylsulfonylisocyanat oder -isothiocyanat der Formel IV

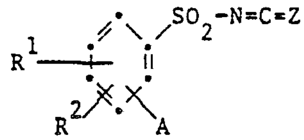

(IV)

worin A, $R^1$, $R^2$ und Z die unter Formel I gegebene Bedeutung haben, gegebenenfalls in Gegenwart einer Base, mit einem Amin der Formel V

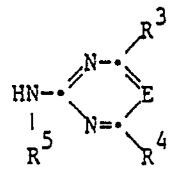

(V)

worin E, $R^3$, $R^4$ und $R^5$ die unter Formel I gegebene Bedeutung haben, umsetzt.

Nach einem weiteren Verfahren werden die Verbindungen der Formel I, in denen $R^5$ Wasserstoff bedeutet, hergestellt, indem man ein Sulfonamid der oben angegebenen Formel II gegebenenfalls in Gegenwart einer Base mit einem Isocyanat oder Isothiocyanat der Formel VI

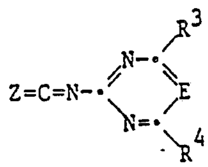

(VI)

worin E, $R^3$, $R^4$, $R^5$ und Z die unter Formel I gegebene Bedeutung haben, umsetzt.

Weiterhin kann man die Verbindungen der Formel I auch erhalten, indem man ein N-Phenylsulfonylcarbamat der Formel VII

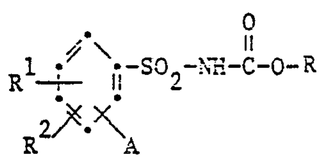

(VII)

worin A, $R^1$ und $R^2$ die unter Formel I gegebene Bedeutung haben und R für Phenyl, Alkyl oder substituiertes Phenyl steht, mit einem Amin der oben angegebenen Formel V umsetzt.

Weiterhin werden die Verbindungen der Formel I, in denen A $C_2$-$C_6$-Halogenalkyl bedeutet, hergestellt, indem man einen Sulfonylharnstoff der Formel VIII

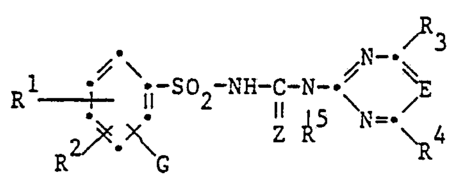

(VIII)

worin $R^1$, $R^2$, $R^3$, $R^4$, $R^5$, E und Z die unter Formel I gegebene Bedeutung haben und G für einen ungesättigten $C_2$-$C_6$-Halogenalkenylrest steht, bis zur Sättigung hydriert oder halogeniert.

Schliesslich werden die Verbindungen der Formel I, in denen A $C_2$-$C_6$-Halogenalkyl bedeutet, auch hergestellt, indem man einen Sulfonylharnstoff der Formel IX

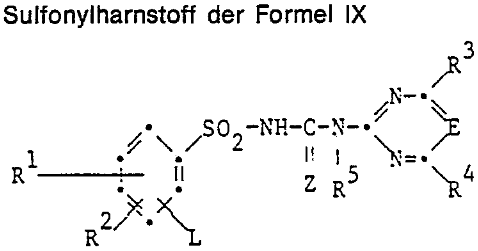

(IX)

worin $R^1$, $R^2$, $R^3$, $R^4$, $R^5$, E und Z die unter Formel I angegebene Bedeutung haben und L für einen ungesättigten $C_2$-$C_6$-Alkenylrest stehen, bis zur Sättigung halogeniert.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzung mit der äquimolaren Menge Base und Verdampfen des Lösungsmittels.

Die Umsetzungen zu Verbindungen der Formel I werden vorteilhafterweise in aprotischen, inerten organischen Lösungsmittel vorgenommen. Solche Lösungsmittel sind Kohlenwasserstoffe wie Benzol, Toluol, Xylol oder Cyclohexan, chlorierte Kohlenwasserstoffe wie Methylenchlorid, Chloroform, Tetrachlorkohlenstoff, oder Chlorbenzol, Aether wie Diäthyläther, Aethylenglykoldimethyläther, Diäthylenglykoldimethyläther, Tetrahydrofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen —20° und + 120 °C. Die Umsetzungen der Kupplungsverfahren verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden. Als Basen sind insbesondere tertiäre Amine wie Trimethylamin, Triäthylamin, Chinuclidin, 1,4-Diazabicyclo-(2,2,2)-octan, 1,5-Diazabicyclo(4,3,0)non-5-en oder 1,5-Diazabicyclo(5,4,3)undec-7-en geeignet.

Die Halogenierungsverfahren werden bei Temperaturen zwischen 0°-100 °C des Reaktionsgemisches durchgeführt. Als vorteilhaft erweist sich hierbei manchmal die Bestrahlung der Reaktionslösung mit Licht oder der Zusatz eines Radikalkettenreaktionsstarters wie Dibenzoylperoxid oder α,α'-Azoisobutyronitril und die Verwendung eines chlorierten Kohlenwasserstoffs wie Methylenchlorid, Chloroform oder Tetrachlorkohlenstoff als Lösungsmittel. Das Hydrierverfahren wird im allgemeinen in einem inerten Lösungsmittel wie Carbonsäuren, Alkohole, Ester oder Kohlenwasserstoffe unter einer Wasserstoffatmosphäre in Gegenwart von Hydrierkatalysatoren wie beispielsweise Raney-Nickel, Palladium oder Platin-Katalysatoren durchgeführt.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Aether, aromatischen Kohlenwasserstoffen oder chlorierten Kohlenwasserstoffen gereinigt werden.

Die Ausgangsmaterialien III, V und VI sind aus der Literatur bekannt oder können nach bekannten Methoden hergestellt werden. Insbesondere können Verbindungen der Formeln III und VI nach bekannten Methoden aus den Verbindungen der Formel V erhalten werden.

Die Ausgangsmaterialien der Formel IX sind teilweise aus der europäischen Patentveröffentlichung 44210 bekannt oder können analog zu dort beschriebenen Methoden synthetisiert werden.

Die Ausgangsmaterialien der Formel VIII und Verfahren zu deren Herstellung sind in der europäischen Patentanmeldung EP-A-102925 beschrieben.

Die Zwischenprodukte der Formeln II, IV und VII sind neu und wurden speziell für die Synthese der Verbindungen der Formel I entwickelt. Sie bilden daher einen Bestandteil der vorliegenden Erfindung.

Die neuen Zwischenprodukte der Formel II können auf verschiedenen Wegen hergestellt werden. So erhält man die Verbindungen der Formel II, indem man Aniline der Formel X

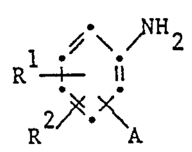

(X)

worin $R^1$, $R^2$ und A die unter Formel I gegebene Bedeutung haben, diazotiert und die Diazogruppe mit Schwefeldioxid in Gegenwart eines Katalysators wie Kupfer-I-chlorid in Salzsäure oder Essigsäure austauscht und das entstandene Phenylsulfonylchlorid mit Ammoniumhydroxid-Lösung umsetzt.

Ebenso kann man die Verbindungen der Formel II erhalten, indem man eine Phenylsulfonsäure der Formel XI

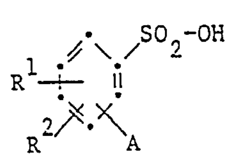

(XI)

worin $R^1$, $R^2$ und A die unter Formel I gegebene Bedeutung haben, durch Behandeln mit einem Chlorierungsmittel wie $PCl_5$, $POCl_3$, $COCl_2$ oder $SOCl_2$ in das entsprechende Phenylsulfonylchlorid

überführt und dieses mit Ammoniumhydroxid-Lösung umsetzt.

Weiter kann man die Verbindungen der Formel II erhalten, indem man einen Benzylthioäther der Formel XII

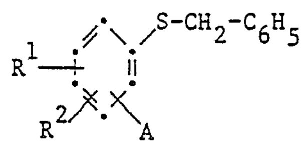

(XII)

worin $R^1$, $R^2$ und A die unter Formel I gegebene Bedeutung haben, durch Behandlung mit Chlor in das entstandene Phenylsulfonylchlorid mit Ammoniumhydroxid-Lösung umsetzt.

Die Verbindungen der Formel II, in denen A für $C_2$-$C_6$-Halogenalkyl steht, können auch hergestellt werden, indem man ein Phenylsulfonamid der Formel XIII

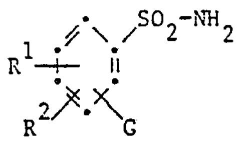

(XIII)

worin $R^1$, $R^2$ und G die unter Formel VIII gegebene Bedeutung haben, bis zur Sättigung hydriert oder halogeniert.

Schliesslich kann man die Verbindungen der Formel II, in denen A für $C_2$-$C_6$-Halogenalkyl steht, auch herstellen, indem man ein Phenylsulfonamid der Formel XIV

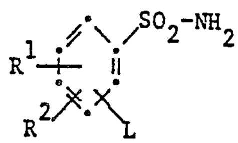

(XIV)

worin $R^1$, $R^2$ und L die unter Formel IX gegebene Bedeutung haben, bis zur Sättigung halogeniert.

Die ebenfalls neuen Phenylsulfonylisocyanate der Formel IV können beispielsweise durch Umsetzungen der Sulfonamide der Formel II mit Phosgen in Anwesenheit von Butylisocyanat in einem chlorierten Kohlenwasserstoff als Lösungsmittel, bei Rückflusstemperatur erhalten werden. Aehnliche Darstellungen sind in « Neuere Methoden der präparativen organischen Chemie », Band IV, 211-229, Verlag Chemie, Weinheim, 1970, beschrieben.

Die Isothiocyanate der Formel IV werden durch Behandlung der Sulfonamide der Formel II mit Schwefelkohlenstoff und Kaliumhydroxid und anschliessende Umsetzung des Dikaliumsalzes mit Phosgen erhalten. Solche Verfahren sind in Arch. Pharm. 299, 174 (1966) beschrieben.

Die N-Phenylsulfonylcarbamate der Formel VII werden durch Umsetzung der Sulfonamide der Formel II mit Diphenylcarbonat in Gegenwart einer Base erhalten. Aehnliche Verfahren sind in der japanischen Patentschrift 61 169 erwähnt.

Die Ausgangsmaterialien der Formeln X, XI und XII sind allgemein bekannt oder sie können nach bekannten Verfahren hergestellt werden.

Die Verbindungen der Formel XIII sind in der europäischen Patentanmeldung EP-A-102925 beschrieben, die der Formel XIV sind aus der europäischen Patentpublikation 44210 bekannt.

Die Verfahren zur Herstellung der Zwischenprodukte verlaufen im allgemeinen unter den gleichen Bedingungen wie sie für die Verfahren zur Herstellung der Endprodukte angegeben sind.

Die Wirkstoffe der Formel I sind stabile Verbindungen. Ihre Handhabung bedarf keiner vorsorglichen Massnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Zuckerrohr, Getreide, Baumwolle, Soja, Mais und Reis befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d. h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei — im Vergleich zu anderen herbiziden und Wuchsregulatoren — sehr geringen Aufwandmengen.

Die Verbindungen der Formel I haben ausserdem starke pflanzenwuchsregulierende Eigenschaften,

die sich in einer Ertragssteigerung von Kulturpflanzen oder Erntegut auswirken kann. Viele Verbindungen der Formel I zeigen darüberhinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden haüfig als « cover crops » (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die « cover crops » jedoch nicht zur Konkurrenz für die Kultur werden können.

Eine Hemmung des vegetativen Wachstums erlaubt bei vielen Kulturpflanzen eine dichtere Anpflanzung der Kultur, so dass eine Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei monokotylen Pflanzen, z. B ; Gräsern oder auch Kulturpflanzen wie Getreide, ist eine Hemmung des vegetativen Wachstums manchmal gewünscht und vorteilhaft. Eine derartige Wuchshemmung ist unter anderem bei Gräsern von wirtschaftlichem Interesse, denn dadurch kann z. B. die Häufigkeit der Grasschnitte in Ziergärten, Park- und Sportanlagen oder an Strassenränden reduziert werden. Von Bedeutung ist auch die Hemmung des Wuchses von krautigen und holzigen Pflanzen an Strassenrändern und in der Nähe von Ueberlandleitungen oder ganz allgemein in Bereichen, in denen ein starker Bewuchs unerwünscht ist.

Wichtig ist auch die Anwendung von Wachstumsregulatoren zur Hemmung des Längenwachstums bei Getreide, denn durch eine Halmverkürzung wird die Gefahr des Umknickens ("Lagerns") der Pflanzen vor der Ernte verringert oder vollkommen beseitigt. Ausserdem können Wachstumsregulatoren oder vollkommen beseitigt. Ausserdem können Wachstumsregulatoren bei Getreide eine Halmverstärkung hervorrufen, die ebenfalls dem Lagern entgegenwirkt.

Weiter eignen sich die Verbindungen der Formel I, um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei grösseren Aufwandmengen werden alle getesteten Pflanzen in ihrer Entwicklung so geschädigt, dass sie absterben.

Die Erfindung betrifft auch herbizide und pflanzenwuchsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmittel eingesetzt und werden daher z. B. zu Emulsionskonzentraten, direkt versprühbaren oder verdünbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyl-taurinsalze zu erwähnen.

Häufig werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazol-derivate enthalten vorzugsweise 2-Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes und Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben :

« Mc Cutcheon's Detergents and Emulsifiers Annual » MC Publishing Corp., Ridgewood, New Jersey, 1981 ;

H. Stache, « Tensid-Taschenbuch », 2.Aufl., C. Hanser Verlag, München, Wien, 1981 ;

M. and J. Ash, « Encyclopedia of Surfactants », Vol. I-III, Chemical Publishing Co., New York, 1980-1981.

Die pestiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen : (% = Gewichtsprozent)

Emulgierbare Konzentrate

| | |
|---|---|
| Aktiver Wirkstoff : | 1 bis 20 %, bevorzugt 5 bis 10 % |
| oberflächenaktive Mittel : | 5 bis 30 %, bevorzugt 10 bis 20 % |
| flüssiges Trägermittel : | 50 bis 94 %, bevorzugt 70 bis 85 % |

Stäube

| | |
|---|---|
| Aktiver Wirkstoff : | 0,1 bis 10 %, vorzugsweise 0,1 bis 1 % |
| festes Trägermittel : | 99,9 bis 90 %, vorzugsweise 99,9 bis 99 % |

Suspensions-Konzentrat

| | |
|---|---|
| Aktiver Wirkstoff : | 5 bis 75 %, vorzugsweise 10 bis 50 % |
| Wasser : | 94 bis 25 %, vorzugsweise 90 bis 30 % |
| oberflächenaktives Mittel : | 1 bis 40 %, vorzugsweise 2 bis 30 % |

Benetzbare Pulver

| | |
|---|---|
| Aktiver Wirkstoff : | 0,5 bis 90 %, vorzugsweise 1 bis 80 % |
| oberflächenaktives Mittel : | 0,5 bis 20 %, vorzugsweise 1 bis 15 % |
| festes Trägermittel : | 5 bis 95 %, vorzugsweise 15 bis 90 % |

Granulate

| | |
|---|---|
| Aktiver Wirkstoff : | 0,5 bis 30 %, vorzugsweise 3 bis 15 % |
| festes Trägermittel : | 99,5 bis 70 %, vorzugsweise 97 bis 85 % |

Während als Handelswerte eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 10 kg/ha, vorzugsweise 0,025 bis 5 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

## Herstellungsbeispiele

Beispiel H1 : N-[2-(3,3,3-Trifluorpropyl)-phenylsulfonyl]-N'-(4,6-dimethoxy-1,3,5-triazin-2-yl)-harnstoff

a) 2-(3,3,3-Trifluorpropyl)-phenylsulfonamid

Eine Mischung von 50,2 g 2-(3,3,3-Trifluor-1-prop-enyl)-phenylsulfonamid, 500 ml Aethylacetat und 5 g eines 5 %igen Pd/C-Katalysators wird für 0,5 Stunden unter einer Wasserstoffatmosphäre bei 20-25° geschüttelt. Durch Abfiltrieren des Katalysators, Eindampfen und Kristallisieren des Rückstandes aus einem Gemisch von Methylenchlorid und Aether erhält man 49,6 g 2-(3,3,3-Trifluorpropyl)-phenylsulfonamid vom Schmelzpunkt 148-149 °C.

b) N-[2-(3,3,3-Trifluorpropyl)-phenylsulfonyl]-N'-methyl-harnstoff

Eine Suspension von 40,5 g 2-(3,3,3,-Trifluor)-phenylsulfonamid in 250 ml absolutem Methylenchlorid wird mit 11,4 g Methylisocyanat versetzt. Anschliessend lässt man 20,2 g Triäthylamin bei 20-25° innerhalb von 15 Minuten zutropfen. Es entsteht eine klare Lösung. Nach Rühren für eine Stunde bei 20-25°, vollständigem Eindampfen, Lösen des Rückstandes in 5 %iger Sodalösung und Ansäuern mit 10 %iger Salzsäure erhält man 46,3 g N-[2-(3,3,3-Trifluorpropyl)-phenylsulfonyl-N'-methylharnstoff vom Schmelzpunkt 176-177 °C.

c) 2-(3,3,3-Trifluorpropyl)-phenylsulfonylisocyanat

40,3 g N-[2-(3,3,3-Trifluorpropyl)-phenylsulfonyl]-N'-methyl-harnstoff werden in 700 ml Chlorbenzol suspendiert und durch azeotrope Abdestillation von ca. 150 ml Lösungsmittel getrocknet. Dann leitet man 71 g Phosgen innerhalb von 90 Minuten bei 120-130° ein. Durch vollständiges Eindampfen der entstandenen Lösung erhält man 37,3 g 2-(3,3,3-Trifluorpropyl)-phenyl-sulfonylisocyanat als gelbliches Oel, das direkt weiter umgesetzt werden kann.

d) N-[2-(3,3,3-Trifluorpropyl)-phenylsulfonyl]-N'-(4,6-dimethoxy-1,3,5-triazin-2-yl)-harnstoff.

5,6 g 2-(3,3,3-Trifluorpropyl)-phenylsulfonyl-isocyanat und 3,12 g 2-Amino-4,6-dimethoxy-1,3,5-triazin werden in 60 ml absolutem Dioxan für 2 Stunden bei 70-80° gerührt. Anschliessend wird das Gemisch mit Aktivkohle behandelt, filtriert und auf ca. 1/5 des Volumens eingedampft. Aus dem Rückstand kristallisieren nach Zugabe von Aether 6,8 g N-[2-(3,3,3-Trifluorpropyl)-phenylsulfonyl]-N'-(4,6-dimethoxy-1,3,5-triazin-2-yl)-harnstoff vom Schmelzpunkt 177-178 °C.

Beispiel H2 : N-[2-(3,3,3-Trifluorpropyl)-phenylsulfonyl[-N'-(4-dimethylamino-6-methoxy-pyrimidin-2-yl)-harnstoff

a) N-[2-(3,3,3-Trifluorpropyl)-phenyl]-phenylcarbamat

Zu einer Suspension von 0,5 g Natriumhydrid (55 %ig) in 10 ml absolutem Dimethylformamid lässt man bei einer Temperatur 15-20° innerhalb von 5 Minuten 2,8 g 2-(3,3,3-Trifluorpropyl)-phenylsulfonamid in 25 ml Dimethylformamid zutropfen und rührt vor dem tropfenweisen Zusatz von 2,5 g Diphenylcarbonat

in 15 ml Dimethylformamid die Suspension für 10 Minuten. Nach weiterem Rühren für 30 Minuten wird das Reaktionsgemisch in eine Mischung aus 100 ml Aethylacetat, 100 g Eis und 10 ml Salzsäure eingegossen. Die organische Phase wird abgetrennt, mit Eiswasser gewaschen, über Natriumsulfat getrocknet und eingedampft. Durch Umkristallisation aus Aether/Petroläther (1 : 1) erhält man 2,8 g N-[2-(3,3,3-Trifluorpropyl)-phenyl]-phenylcarbamat vom Schmelzpunkt 116-119 °C.

b) N-[2-(3,3,3-Trifluorpropyl)-phenylsulfonyl]-N'-(4-dimethylamino-6-methoxy-pyrimidin-2-yl)-harnstoff

Eine Mischung von 2,65 g N-[2-(3,3,3-Trifluorpropyl)-phenyl]-phenylcarbamat und 1,18 g 2-Amino-4-dimethylamino-6-methoxy-pyrimidin wird in 20 ml absolutem Dioxan für 90 Minuten zum Rückfluss erhitzt, danach auf 20° abgekühlt und eingedampft. Durch Umkristallisieren aus einem Gemisch von Aceton und Aether erhält man 1,65 g N-[2-(3,3,3-Trifluorpropyl)-phenylsulfonyl]-N'-(4-dimethylamino-6-methoxy-pyrimidin-2-yl)-harnstoff vom Schmelzpunkt 183-184 °C.

Beispiel H3 : N-[2-(3,3,3-Trifluorpropyl)-phenylsulfonyl]-N'-(4-methoxy-6-methyl-1,3,5-triazin-2-yl)-harnstoff

2,9 g N-[2-(3,3,3-Trifluor-1-propenyl)-phenylsulfonyl]-N'-(4-methoxy-6-methyl-1,3,5-triazin-2-yl)-harnstoff werden in 50 ml Essigester gelöst, mit 1,5 g eines 5 %igen Palladium/Kohle-Katalysators versetzt und für 16 Stunden bei 20-25° in einer Wasserstoffatmosphäre geschüttelt. Durch Abfiltrieren des Katalysators, vollständiges Eindampfen und Kristallisieren des Rückstandes aus einem Aceton/Aether-Gemisch erhält man 2,2 g N-[2-(3,3,3-Trifluorpropyl)-phenylsulfonyl]-N'-(4-methoxy-6-methyl-1,3,5-triazin-2-yl)-harnstoff vom Schmelzpunkt 157-158 °C.

Beispiel H4 : N-[2-(3,3,3-Trifluorpropyl)-phenylsulfonyl]-N'-(4-methoxy-6-methylpyrimidin-2-yl)-harnstoff

Eine Mischung von 2,53 g 2-(3,3,3-Trifluorpropyl)-phenylsulfonamid, 2,59 g 4-Methoxy-6-methyl-pyrimidin-2-yl-phenylcarbamat, 1,52 g 1,5-Diazabicyclo (5.4.0) undec-5-en und 20 ml Dioxan wird 3 Stunden bei 20-25° Raumtemperatur gerührt. Durch Eingiessen in Wasser, Ansäuern mit 10 %iger Salzsäure, Extraktion mit Aethylacetat, Trocknen der organischen Phase über Natriumsulfat, vollständigem Eindampfen und Umkristallisieren des Rückstandes aus Aceton/Aether-Gemisch (1 : 5) erhält man 3,5 g N-[2-(3,3,3-Trifluorpropyl)-phenylsulfonyl]-N'-(4-methoxy-6-methyl-pyrimidin-2-yl)-harnstoff vom Schmelzpunkt 174-175 °C.

In analoger Weise werden die in den folgenden Tabellen aufgelisteten Zwischen- und Endprodukte hergestellt.

Siehe Tabellen I-II Seite 11 ff.)

Tabelle I

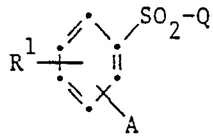

| Nr. | A | Stellung von A | $R^1$ | Q | Phys. Daten |
|---|---|---|---|---|---|
| 1.1 | $-CH_2-CH_2-CF_3$ | 2 | H | Cl | |
| 1.2 | $-CH_2-CH_2-CF_3$ | 2 | H | $NH_2$ | Smp. 148-149 °C |
| 1.3 | $-CH_2-CH_2-CF_3$ | 2 | H | $-N=C=O$ | Oe1 |
| 1.4 | $-CH_2-CH_2-CF_3$ | 2 | H | $-NH-CO-OC_6H_5$ | Smp. 116-119 °C |
| 1.5 | $CH_2F$ | 2 | H | Cl | |
| 1.6 | $CH_2F$ | 2 | H | $NH_2$ | |
| 1.7 | $CH_2F$ | 2 | H | $-N=C=O$ | |
| 1.8 | $CH_2F$ | 2 | H | $-NH-CO-OC_6H_5$ | |
| 1.9 | $CHF_2$ | 2 | H | Cl | |
| 1.10 | $CHF_2$ | 2 | H | $NH_2$ | |
| 1.11 | $CHF_2$ | 2 | H | $-N=C=O$ | |
| 1.12 | $CHF_2$ | 2 | H | $-NH-CO-OC_6H_5$ | |
| 1.13 | $-CH_2-CH_2-C_3F_7-n$ | 2 | H | Cl | |
| 1.14 | $-CH_2-CH_2-C_3F_7-n$ | 2 | H | $NH_2$ | |
| 1.15 | $-CH_2-CH_2-C_3F_7-n$ | 2 | H | $-N=C=O$ | |
| 1.16 | $-CH_2-CH_2-C_3F_7-n$ | 2 | H | $-NH-CO-OC_6H_5$ | |
| 1.17 | $-CH_2-CH(CF_3)-CH_2-CF_3$ | 2 | H | Cl | |
| 1.18 | $-CH_2-CH(CF_3)-CH_2-CF_3$ | 2 | H | $NH_2$ | |
| 1.19 | $-CH_2-CH(CF_3)-CH_2-CF_3$ | 2 | H | $-N=C=O$ | |
| 1.20 | $-CH_2-CH(CF_3)-CH_2-CF_3$ | 2 | H | $-NH-CO-OC_6H_5$ | |
| 1.21 | CHClF | 2 | H | Cl | |
| 1.22 | CHClF | 2 | H | $NH_2$ | |
| 1.23 | CHClF | 2 | H | $-N=C=O$ | |
| 1.24 | CHClF | 2 | H | $-NH-CO-OC_6H_5$ | |
| 1.25 | $-CH_2-CH_2-CF_2-CH_3$ | 2 | H | Cl | |
| 1.26 | $-CH_2-CH_2-CF_2-CH_3$ | 2 | H | $NH_2$ | |
| 1.27 | $-CH_2-CH_2-CF_2-CH_3$ | 2 | H | $-N=C=O$ | |
| 1.28 | $-CH_2-CH_2-CF_2-CH_3$ | 2 | H | $-NH-CO-OC_6H_5$ | |
| 1.29 | $-CH_2-CH_2-CF_3$ | 3 | H | Cl | |
| 1.30 | $-CH_2-CH_2-CF_3$ | 3 | H | $NH_2$ | |
| 1.31 | $-CH_2-CH_2-CF_3$ | 3 | H | $-N=C=O$ | |
| 1.32 | $-CH_2-CH_2-CF_3$ | 3 | H | $-NH-CO-OC_6H_5$ | |
| 1.33 | $-CHCl-CH_2Cl$ | 2 | H | Cl | |
| 1.34 | $-CHCl-CH_2Cl$ | 2 | H | $NH_2$ | |
| 1.35 | $-CHCl-CH_2Cl$ | 2 | H | $-N=C=O$ | |
| 1.36 | $-CHCl-CH_2Cl$ | 2 | H | $-NH-CO-OC_6H_5$ | |
| 1.37 | $-CHCl-CHCl-CH_3$ | 2 | H | Cl | |
| 1.38 | $-CHCl-CHCl-CH_3$ | 2 | H | $NH_2$ | |
| 1.39 | $-CHCl-CHCl-CH_3$ | 2 | H | $-N=C=O$ | |
| 1.40 | $-CHCl-CHCl-CH_3$ | 2 | H | $-NH-CO-OC_6H_5$ | |
| 1.41 | $-CHCl-CHCl-CF_3$ | 2 | H | Cl | |
| 1.42 | $-CHCl-CHCl-CF_3$ | 2 | H | $NH_2$ | |
| 1.43 | $-CHCl-CHCl-CF_3$ | 2 | H | $-N=C=0$ | |
| 1.44 | $-CHCl-CHCl-CF_3$ | 2 | H | $-NH-CO-OC_6H_5$ | |
| 1.45 | $-CH_2-CH_2-CH_2-Cl$ | 2 | H | Cl | |
| 1.46 | $-CH_2-CH_2-CH_2-Cl$ | 2 | H | $NH_2$ | |
| 1.47 | $-CH_2-CH_2-CH_2-Cl$ | 2 | H | $-N=C=O$ | |
| 1.48 | $-CH_2-CH_2-CH_2-Cl$ | 2 | H | $-NH-CO-OC_6H_5$ | |
| 1.49 | $-CH_2-CH_2-CH_2F$ | 2 | H | Cl | |
| 1.50 | $-CH_2-CH_2-CH_2F$ | 2 | H | $NH_2$ | |
| 1.51 | $-CH_2-CH_2-CH_2F$ | 2 | H | $-N=C=O$ | |
| 1.52 | $-CH_2-CH_2-CH_2F$ | 2 | H | $-NH-CO-OC_6H_5$ | |

Tabelle 2

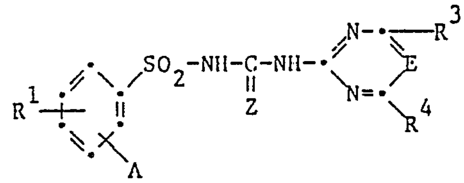

| Nr. | A | Stellung von A | $R^1$ | $R^3$ | $R^4$ | E | Z | Smp. [°C] |
|---|---|---|---|---|---|---|---|---|
| 2.1 | $-CH_2-CH_2-C_3F_9-n$ | 2 | H | $CH_3$ | $OCH_3$ | N | O | 150-151 |
| 2.2 | $-CH_2-CH_2-C_3F_9-n$ | 2 | H | $OCH_3$ | $OCH_3$ | N | O | |
| 2.3 | $-CH_2-CH_2-C_3F_9-n$ | 2 | H | $CH_3$ | $OC_2H_5$ | N | O | |
| 2.4 | $-CH_2-CH_2-C_3F_9-n$ | 2 | H | $C_2H_5$ | $OCH_3$ | N | O | |
| 2.5 | $-CH_2-CH_2-C_3F_9-n$ | 2 | H | $OCH_3$ | $-N(CH_3)_2$ | N | O | |
| 2.6 | $-CH_2-CH_2-C_3F_9-n$ | 2 | H | $OCH_3$ | $-NHCH_3$ | N | O | |
| 2.7 | $-CH_2-CH_2-C_3F_9-n$ | 2 | H | $OCH_3$ | $-OCH_2-CF_3$ | N | O | |
| 2.8 | $-CH_2-CH(CF_3)-CH_2-CF_3$ | 2 | H | $CH_3$ | $OCH_3$ | N | O | 167-169 |
| 2.9 | $-CH_2-CH(CF_3)-CH_2-CF_3$ | 2 | H | $OCH_3$ | $OCH_3$ | N | O | |
| 2.10 | $-CH_2-CH(CF_3)-CH_2-CF_3$ | 2 | H | $CH_3$ | $OC_2H_5$ | N | O | |
| 2.11 | $-CH_2-CH(CF_3)-CH_2-CF_3$ | 2 | H | $C_2H_5$ | $OCH_3$ | N | O | |
| 2.12 | $-CH_2-CH(CF_3)-CH_2-CF_3$ | 2 | H | $OCH_3$ | $-N(CH_3)_2$ | N | O | |
| 2.13 | $-CH_2-CH(CF_3)-CH_2-CF_3$ | 2 | H | $OCH_3$ | $-NHCH_3$ | N | O | |
| 2.14 | $-CH_2-CH(CF_3)-CH_2-CF_3$ | 2 | H | $OCH_3$ | $-OCH_2-CF_3$ | N | O | |
| 2.15 | $-CH_2-CH_2-CH_2Cl$ | 2 | H | $OCH_3$ | $CH_3$ | N | O | |
| 2.16 | $-CH_2-CH_2-CH_2Cl$ | 2 | H | $OCH_3$ | $OCH_3$ | N | O | |
| 2.17 | $-CH_2-CH_2-CH_2Cl$ | 2 | H | $CH_3$ | $OC_2H_5$ | N | O | |
| 2.18 | $-CH_2-CH_2-CH_2Cl$ | 2 | H | $C_2H_5$ | $OCH_3$ | N | O | |
| 2.19 | $-CH_2-CH_2-CH_2Cl$ | 2 | H | $OCH_3$ | $-N(CH_3)_2$ | N | O | |
| 2.20 | $-CH_2-CH_2-CH_2Cl$ | 2 | H | $OCH_3$ | $-NHCH_3$ | N | O | |
| 2.21 | $-CH_2-CH_2-CH_2Cl$ | 2 | H | $OCH_3$ | $-OCH_2-CF_3$ | N | O | |
| 2.22 | $-CH_2-CH_2-CH_2F$ | 2 | H | $OCH_3$ | $CH_3$ | N | O | |
| 2.23 | $-CH_2-CH_2-CH_2F$ | 2 | H | $OCH_3$ | $OCH_3$ | N | O | |
| 2.24 | $-CH_2-CH_2-CH_2F$ | 2 | H | $OCH_3$ | $C_2H_5$ | N | O | |
| 2.25 | $-CH_2-CH_2-CH_2F$ | 2 | H | $CH_3$ | $OC_2H_5$ | N | O | |
| 2.26 | $-CH_2-CH_2-CH_2F$ | 2 | H | $OCH_3$ | $-N(CH_3)_2$ | N | O | |
| 2.27 | $-CH_2-CH_2-CH_2F$ | 2 | H | $OCH_3$ | $-NHCH_3$ | N | O | |
| 2.28 | $-CH_2-CH_2-CH_2F$ | 2 | H | $OCH_3$ | $-OCH_2-CF_3$ | N | O | |
| 2.29 | $-CHClF$ | 2 | H | $CH_3$ | $OCH_3$ | N | O | |
| 2.30 | $CHClF$ | 2 | H | $OCH_3$ | $OCH_3$ | N | O | |
| 2.31 | $CHClF$ | 2 | H | $CH_3$ | $OC_2H_5$ | N | O | |
| 2.32 | $CHClF$ | 2 | H | $C_2H_5$ | $OCH_3$ | N | O | |
| 2.33 | $CHClF$ | 2 | H | $C_2H_5$ | $-N(CH_3)_2$ | N | O | |
| 2.34 | $CHClF$ | 2 | H | $OCH_3$ | $-NHCH_3$ | N | O | |
| 2.35 | $CHClF$ | 2 | H | $OCH_3$ | $-OCH_2-CF_3$ | N | O | |
| 2.36 | $CHBrF$ | 2 | H | $CH_3$ | $OCH_3$ | N | O | |
| 2.37 | $CHBrF$ | 2 | H | $OCH_3$ | $OCH_3$ | N | O | |
| 2.38 | $CHBrF$ | 2 | H | $CH_3$ | $OC_2H_5$ | N | O | |
| 2.39 | $CHBrF$ | 2 | H | $C_2H_5$ | $OCH_3$ | N | O | |
| 2.40 | $CHBrF$ | 2 | H | $OCH_3$ | $-N(CH_3)_2$ | N | O | |
| 2.41 | $CHBrF$ | 2 | H | $OCH_3$ | $-NHCH_3$ | N | O | |
| 2.42 | $CHBrF$ | 2 | H | $OCH_3$ | $-OCH_2-CF_3$ | N | O | |
| 2.42 a | $CH_2F$ | 2 | H | $CH_3$ | $OCH_3$ | N | O | |
| 2.43 | $CH_2F$ | 2 | H | $OCH_3$ | $OCH_3$ | N | O | |
| 2.44 | $CH_2F$ | 2 | H | $C_2H_5$ | $OCH_3$ | N | O | |
| 2.45 | $CH_2F$ | 2 | H | $OCH_3$ | $-N(CH_3)_2$ | N | 0 | |
| 2.46 | $-CH_2F$ | 2 | H | $OCH_3$ | $-NHCH_3$ | N | O | |
| 2.47 | $CH_2F$ | 2 | H | $OCH_3$ | $-OCH_2-CF_3$ | N | O | |
| 2.48 | $CH_2F$ | 2 | H | $CH_3$ | $OC_2H_5$ | N | O | |
| 2.49 | $CHF_2$ | 2 | H | $CH_3$ | $OCH_3$ | N | O | |
| 2.50 | $CHF_2$ | 2 | H | $OCH_3$ | $OCH_3$ | N | O | |
| 2.51 | $CHF_2$ | 2 | H | $C_2H_5$ | $OCH_3$ | N | O | |

Tabelle 2 (Fortsetzung)

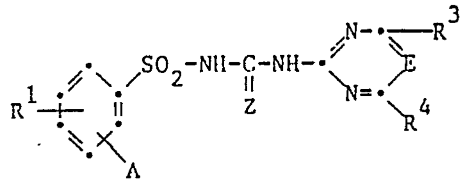

| Nr. | A | Stellung von A | $R^1$ | $R^3$ | $R^4$ | E | Z | Smp. [°C] |
|---|---|---|---|---|---|---|---|---|
| 2.52 | $CHF_2$ | 2 | H | $OCH_3$ | $-N(CH_3)_2$ | N | O | |
| 2.53 | $CHF_2$ | 2 | H | $OCH_3$ | $-NHCH_3$ | N | O | |
| 2.54 | $CHF_2$ | 2 | H | $OCH_3$ | $-OCH_2-CF_3$ | N | O | |
| 2.55 | $CHF_2$ | 2 | H | $CH_3$ | $OC_2H_5$ | N | O | |
| 2.56 | $-CH_2-CH_2-CF_3$ | 2 | H | $CH_3$ | $OCH_3$ | N | O | 157-158 |
| 2.57 | $-CH_2-CH_2-CF_3$ | 2 | H | $OCH_3$ | $OCH_3$ | N | O | 177-178 |
| 2.58 | $-CH_2-CH_2-CF_3$ | 2 | H | $CH_3$ | $OC_2H_5$ | N | O | 145-146 |
| 2.59 | $-CH_2-CH_2-CF_3$ | 2 | H | $C_2H_5$ | $OCH_3$ | N | O | |
| 2.60 | $-CH_2-CH_2-CF_3$ | 2 | H | $C_2H_5$ | $OC_2H_5$ | N | O | |
| 2.61 | $-CH_2-CH_2-CF_3$ | 2 | H | $OCH_3$ | $-OCH_2-CF_3$ | N | O | |
| 2.62 | $-CH_2-CH_2-CF_3$ | 2 | H | $CH_3$ | $-OCH_2-CF_3$ | N | O | |
| 2.63 | $-CH_2-CH_2-CF_3$ | 2 | H | $OCH_3$ | $-N(CH_3)_2$ | N | O | |
| 2.64 | $-CH_2-CH_2-CF_3$ | 2 | H | $OCH_3$ | $OC_3H_7-i$ | N | O | |
| 2.65 | $-CH_2-CH_2-CF_3$ | 2 | H | $SCH_3$ | $OCH_3$ | N | O | |
| 2.66 | $-CH_2-CH_2-CF_3$ | 2 | H | $OCH_3$ | $-NHCH_3$ | N | O | |
| 2.67 | $-CH_2-CH_2-CF_3$ | 2 | H | $CH_3$ | $CH_3$ | N | O | |
| 2.68 | $-CH_2-CH_2-CF_3$ | 2 | H | $SCHF_2$ | $OCH_3$ | N | O | |
| 2.69 | $-CH_2-CH_2-CF_2-CH_3$ | 2 | H | $CH_3$ | $OCH_3$ | N | O | 150-152 |
| 2.70 | $-CH_2-CH_2-CF_2-CH_3$ | 2 | H | $OCH_3$ | $OCH_3$ | N | O | |
| 2.71 | $-CH_2-CH_2-CF_2-CH_3$ | 2 | H | $CH_3$ | $OC_2H_5$ | N | O | |
| 2.72 | $-CH_2-CH_2-CF_2-CH_3$ | 2 | H | $C_2H_5$ | $OCH_3$ | N | O | |
| 2.73 | $-CH_2-CH_2-CF_2-CH_3$ | 2 | H | $OCH_3$ | $-N(CH_3)_2$ | N | O | |
| 2.74 | $-CH_2-CH_2-CF_2-CH_3$ | 2 | H | $OCH_3$ | $-NHCH_3$ | N | O | |
| 2.75 | $-CH_2-CH_2-CF_2-CH_3$ | 2 | H | $OCH_3$ | $-OCH_2-CF_3$ | N | O | |
| 2.76 | $-CH_2-CH_2-CF_3$ | 3 | H | $CH_3$ | $OCH_3$ | N | O | |
| 2.77 | $-CH_2-CH_2-CF_3$ | 3 | H | $OCH_3$ | $OCH_3$ | N | O | |
| 2.78 | $-CH_2-CH_2-CF_3$ | 3 | H | $CH_3$ | $OC_2H_5$ | N | O | |
| 2.79 | $-CH_2-CH_2-CF_3$ | 3 | H | $C_2H_5$ | $OCH_3$ | N | O | |
| 2.80 | $-CH_2-CH_2-CF_3$ | 3 | H | $OCH_3$ | $-N(CH_3)_2$ | N | O | |
| 2.81 | $-CH_2-CH_2-CF_3$ | 3 | H | $OCH_3$ | $-NHCH_3$ | N | O | |
| 2.82 | $-CH_2-CH_2-CF_3$ | 3 | H | $OCH_3$ | $-OCH_2-CF_3$ | N | O | |
| 2.83 | $-CHCl-CH_2Cl$ | 2 | H | $CH_3$ | $OCH_3$ | N | O | |
| 2.84 | $-CHCl-CH_2Cl$ | 2 | H | $OCH_3$ | $OCH_3$ | N | O | |
| 2.85 | $-CHCl-CH_2Cl$ | 2 | H | $C_2H_5$ | $OCH_3$ | N | O | |
| 2.86 | $-CHCl-CH_2Cl$ | 2 | H | $CH_3$ | $OC_2H_5$ | N | O | |
| 2.87 | $-CHCl-CH_2Cl$ | 2 | H | $OCH_3$ | $-(CH_3)_2$ | N | O | |
| 2.88 | $-CHCl-CH_2Cl$ | 2 | H | $OCH_3$ | $-NHCH_3$ | N | O | |
| 2.89 | $-CHCl-CH_2Cl$ | 2 | H | $OCH_3$ | $-OCH_2-CF_3$ | N | O | |
| 2.90 | $-CHCl-CHCl-CH_3$ | 2 | H | $OCH_3$ | $CH_3$ | N | O | |
| 2.91 | $-CHCl-CHCl-CH_3$ | 2 | H | $OCH_3$ | $C_2H_5$ | N | O | |
| 2.92 | $-CHCl-CHCl-CH_3$ | 2 | H | $OCH_3$ | $OCH_3$ | N | O | |
| 2.93 | $-CHCl-CHCl-CH_3$ | 2 | H | $CH_3$ | $OC_2H_5$ | N | O | |
| 2.94 | $-CHCl-CHCl-CH_3$ | 2 | H | $OCH_3$ | $-N(CH_3)_2$ | N | O | |
| 2.95 | $-CHCl-CHCl-CH_3$ | 2 | H | $OCH_3$ | $-NHCH_3$ | N | O | |
| 2.96 | $-CHCl-CHCl-CH_3$ | 2 | H | $OCH_3$ | $-OCH_2-CF_3$ | N | O | |
| 2.97 | $-CH_2-CH_2-CF_3$ | 2 | H | $CH_3$ | $CH_3$ | CH | O | |
| 2.98 | $-CH_2-CH_2-CF_3$ | 2 | H | $CH_3$ | $OCH_3$ | CH | O | 174-175 |
| 2.99 | $-CH_2-CH_2-CF_3$ | 2 | H | $CH_3$ | $-OCH_2-CF_3$ | CH | O | |

Tabelle 2 (Fortsetzung)

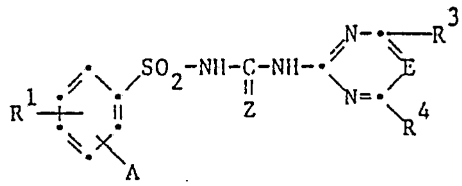

| Nr. | A | Stellung von A | $R^1$ | $R^3$ | $R^4$ | E | Z | Smp. [°C] |
|---|---|---|---|---|---|---|---|---|
| 2.100 | $-CH_2-CH_2-CF_3$ | 2 | H | $CH_3$ | $OC_2H_5$ | CH | O | |
| 2.101 | $-CH_2-CH_2-CF_3$ | 2 | H | $OCH_3$ | Cl | CH | O | 191-192 |
| 2.102 | $-CH_2-CH_2-CF_3$ | 2 | H | $OCH_3$ | $OCH_3$ | CH | O | 182-183 |
| 2.103 | $-CH_2-CH_2-CF_3$ | 2 | H | $OCH_3$ | $SCHF_2$ | CH | O | |
| 2.104 | $-CH_2-CH_2-CF_3$ | 2 | H | $-OCH_3$ | $-N(CH_3)_2$ | CH | O | 183-184 |
| 2.105 | $-CH_2-CH_2-CF_3$ | 2 | H | $OCH_3$ | $-NHCH_3$ | CH | O | |
| 2.106 | $-CH_2-CH_2-CF_3$ | 2 | H | $OCH_3$ | $CH_2F$ | CH | O | |
| 2.107 | $-CH_2-CH_2-CF_3$ | 2 | H | $OCH_3$ | $-CF_3$ | CH | O | |
| 2.108 | $-CH_2-CH_2-CF_3$ | 2 | H | $OCH_3$ | $SCH_3$ | CH | O | |
| 2.109 | $-CH_2-CH_2-CF_3$ | 2 | H | $OCH_3$ | $-OCH_2-CF_3$ | CH | O | |
| 2.110 | $-CH_2-CH_2-CF_3$ | 2 | H | $OCH_3$ | $-CH_2-OCH_3$ | CH | O | |
| 2.111 | $-CH_2-CH_2-CF_3$ | 2 | H | $OCH_3$ | $-CH_2-OC_2H_5$ | CH | O | |
| 2.112 | $CH_2F$ | 2 | H | $CH_3$ | $OCH_3$ | CH | O | |
| 2.113 | $CH_2F$ | 2 | H | $CH_3$ | $CH_3$ | CH | O | |
| 2.114 | $CH_2F$ | 2 | H | $OCH_3$ | Cl | CH | O | |
| 2.115 | $CH_2F$ | 2 | H | $OCH_3$ | $-N(CH_3)_2$ | CH | O | |
| 2.116 | $CH_2F$ | 2 | H | $OCH_3$ | $CH_2F$ | CH | O | |
| 2.117 | $CH_2F$ | 2 | H | $OCH_3$ | $-CH_2-OCH_3$ | CH | O | |
| 2.118 | $CH_2F$ | 2 | H | $OCH_3$ | $OCH_3$ | CH | O | |
| 2.119 | $CHF_2$ | 2 | H | $CH_3$ | $OCH_3$ | CH | O | |
| 2.120 | $CHF_2$ | 2 | H | $CH_3$ | $CH_3$ | CH | O | |
| 2.121 | $CHF_2$ | 2 | H | $OCH_3$ | Cl | CH | O | |
| 2.122 | $CHF_2$ | 2 | H | $OCH_3$ | $-N(CH_3)_2$ | CH | O | |
| 2.123 | $CHF_2$ | 2 | H | $OCH_3$ | $CH_2F$ | CH | O | |
| 2.124 | $CHF_2$ | 2 | H | $OCH_3$ | $-CH_2-OCH_3$ | CH | O | |
| 2.125 | $CHF_2$ | 2 | H | $OCH_3$ | $OCH_3$ | CH | O | |
| 2.126 | $-CHCl-CH_2Cl$ | 2 | H | $CH_3$ | $OCH_3$ | CH | O | |
| 2.127 | $-CHCl-CH_2Cl$ | 2 | H | $CH_3$ | $CH_3$ | CH | O | |
| 2.128 | $-CHCl-CH_2Cl$ | 2 | H | $OCH_3$ | Cl | CH | O | |
| 2.129 | $-CHCl-CH_2Cl$ | 2 | H | $OCH_3$ | $-N(CH_3)_2$ | CH | O | |
| 2.130 | $-CHCl-CH_2Cl$ | 2 | H | $OCH_3$ | $CH_2F$ | CH | O | |
| 2.131 | $-CHCl-CH_2Cl$ | 2 | H | $OCH_3$ | $-CH_2-OCH_3$ | CH | O | |
| 2.132 | $-CHCl-CH_2Cl$ | 2 | H | $OCH_3$ | $OCH_3$ | CH | O | |
| 2.133 | $-CHCl-CHCl-CH_3$ | 2 | H | $OCH_3$ | $CH_3$ | CH | O | |
| 2.134 | $-CHCl-CHCl-CH_3$ | 2 | H | $CH_3$ | $CH_3$ | CH | O | |
| 2.135 | $-CHCl-CHCl-CH_3$ | 2 | H | $OCH_3$ | Cl | CH | O | |
| 2.136 | $-CHCl-CHCl-CH_3$ | 2 | H | $OCH_3$ | $-N(CH_3)_2$ | CH | O | |
| 2.137 | $-CHCl-CHCl-CH_3$ | 2 | H | $OCH_3$ | $CH_2F$ | CH | O | |
| 2.138 | $-CHCl-CHCl-CH_3$ | 2 | H | $OCH_3$ | $-CH_2-OCH_3$ | CH | O | |
| 2.139 | $-CHCl-CHCl-CH_3$ | 2 | H | $OCH_3$ | $OCH_3$ | CH | O | |
| 2.140 | $-CH_2-CH_2-CF_2-CH_3$ | 2 | H | $CH_3$ | $OCH_3$ | CH | O | |
| 2.141 | $-CH_2-CH_2-CF_2-CH_3$ | 2 | H | $CH_3$ | $OCH_3$ | CH | O | |
| 2.142 | $-CH_2-CH_2-CF_2-CH_3$ | 2 | H | $OCH_3$ | Cl | CH | O | |
| 2.143 | $-CH_2-CH_2-CF_2-CH_3$ | 2 | H | $OCH_3$ | $-N(CH_3)_2$ | CH | O | |
| 2.144 | $-CH_2-CH_2-CF_2-CH_3$ | 2 | H | $OCH_3$ | $CH_2F$ | CH | O | |
| 2.145 | $-CH_2-CH_2-CF_2-CH_3$ | 2 | H | $OCH_3$ | $CH_2F$ | CH | O | |
| 2.146 | $-CH_2-CH_2-CF_2-CH_3$ | 2 | H | $OCH_3$ | $-CH_2-OCH_3$ | CH | O | |
| 2.147 | $-CH_2-CH_2-CF_2-CH_3$ | 2 | H | $OCH_3$ | $OCH_3$ | CH | O | |
| 2.148 | $-CH_2-CH_2-CF_3$ | 3 | H | $OCH_3$ | $CH_3$ | CH | O | |
| 2.149 | $-CH_2-CH_2-CF_3$ | 3 | H | $CH_3$ | $CH_3$ | CH | O | |
| 2.150 | $-CH_2-CH_2-CF_3$ | 3 | H | $OCH_3$ | Cl | CH | O | |
| 2.151 | $-CH_2-CH_2-CF_3$ | 3 | H | $OCH_3$ | $-N(CH_3)_2$ | CH | O | |

Tabelle 2 (Fortsetzung)

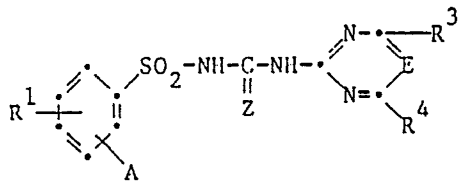

| Nr. | A | Stellung von A | $R^1$ | $R^3$ | $R^4$ | E | Z | Smp. [°C] |
|---|---|---|---|---|---|---|---|---|
| 2.152 | $-CH_2-CH_2-CF_3$ | 3 | H | $OCH_3$ | $CH_2F$ | CH | O | |
| 2.153 | $-CH_2-CH_2-CF_3$ | 3 | H | $OCH_3$ | $-CH_2-OCH_3$ | CH | O | |
| 2.154 | $-CH_2-CH_2-CF_3$ | 3 | H | $OCH_3$ | $OCH_3$ | CH | O | |
| 2.155 | $-CH_2-CH_2-CH_2Cl$ | 2 | H | $CH_3$ | $OCH_3$ | CH | O | |
| 2.156 | $-CH_2-CH_2-CH_2Cl$ | 2 | H | $CH_3$ | $CH_3$ | CH | O | |
| 2.157 | $-CH_2-CH_2-CH_2Cl$ | 2 | H | $OCH_3$ | Cl | CH | O | |
| 2.158 | $-CH_2-CH_2-CH_2Cl$ | 2 | H | $OCH_3$ | $-N(CH_3)_2$ | CH | O | |
| 2.159 | $-CH_2-CH_2-CH_2Cl$ | 2 | H | $OCH_3$ | $CH_2F$ | CH | O | |
| 2.160 | $-CH_2-CH_2-CH_2Cl$ | 2 | H | $OCH_3$ | $-CH_2-OCH_3$ | CH | O | |
| 2.161 | $-CH_2-CH_2-CH_2Cl$ | 2 | H | $OCH_3$ | $OCH_3$ | CH | O | |
| 2.162 | $-CH_2-CH_2-CH_2F$ | 2 | H | $OCH_3$ | $CH_3$ | CH | O | |
| 2.163 | $-CH_2-CH_2-CH_2F$ | 2 | H | $CH_3$ | $CH_3$ | CH | O | |
| 2.164 | $-CH_2-CH_2-CH_2F$ | 2 | H | $OCH_3$ | Cl | CH | O | |
| 2.165 | $-CH_2-CH_2-CH_2F$ | 2 | H | $OCH_3$ | $-N(CH_3)_2$ | CH | O | |
| 2.166 | $-CH_2-CH_2-CH_2F$ | 2 | H | $OCH_3$ | $CH_2F$ | CH | O | |
| 2.167 | $-CH_2-CH_2-CH_2F$ | 2 | H | $OCH_3$ | $-CH_2-OCH_3$ | CH | O | |
| 2.168 | $-CH_2-CH_2-CH_2F$ | 2 | H | $OCH_3$ | $OCH_3$ | CH | O | |
| 2.169 | CHClF | 2 | H | $OCH_3$ | $CH_3$ | CH | O | |
| 2.170 | CHClF | 2 | H | $CH_3$ | $CH_3$ | CH | O | |
| 2.171 | CHClF | 2 | H | $OCH_3$ | Cl | CH | O | |
| 2.172 | CHClF | 2 | H | $OCH_3$ | $-N(CH_3)_2$ | CH | O | |
| 2.173 | CHClF | 2 | H | $OCH_3$ | $CH_2F$ | CH | O | |
| 2.174 | CHClF | 2 | H | $OCH_3$ | $-CH_2-OCH_3$ | CH | O | |
| 2.175 | CHClF | 2 | H | $OCH_3$ | $OCH_3$ | CH | O | |
| 2.176 | CHBrF | 2 | H | $OCH_3$ | $CH_3$ | CH | O | |
| 2.177 | CHBrF | 2 | H | $CH_3$ | $CH_3$ | CH | O | |
| 2.178 | CHBrF | 2 | H | $OCH_3$ | Cl | CH | O | |
| 2.179 | CHBrF | 2 | H | $OCH_3$ | $-N(CH_3)_2$ | CH | O | |
| 2.180 | CHBrF | 2 | H | $OCH_3$ | $CH_2F$ | CH | O | |
| 2.181 | CHBrF | 2 | H | $OCH_3$ | $-CH_2-OCH_3$ | CH | O | |
| 2.182 | CHBrF | 2 | H | $OCH_3$ | $OCH_3$ | CH | O | |
| 2.183 | $-CH_2-CH_2-C_3F_7-n$ | 2 | H | $CH_3$ | $OCH_3$ | CH | O | |
| 2.184 | $-CH_2-CH_2-C_3F_7-n$ | 2 | H | $CH_3$ | $CH_3$ | CH | O | |
| 2.185 | $-CH_2-CH_2-C_3F_7-n$ | 2 | H | $OCH_3$ | Cl | CH | O | |
| 2.186 | $-CH_2-CH_2-C_3F_7-n$ | 2 | H | $OCH_3$ | $-N(CH_3)_2$ | CH | O | |
| 2.187 | $-CH_2-CH_2-C_3F_7-n$ | 2 | H | $OCH_3$ | $CH_2F$ | CH | O | |
| 2.188 | $-CH_2-CH_2-C_3F_7-n$ | 2 | H | $OCH_3$ | $-CH_2-OCH_3$ | CH | O | |
| 2.189 | $-CH_2-CH_2-C_3F_7-n$ | 2 | H | $OCH_3$ | $OCH_3$ | CH | O | |
| 2.190 | $-CH_2-CH(CF_3)-CH_2-CF_3$ | 2 | H | $CH_3$ | $OCH_3$ | CH | O | |
| 2.191 | $-CH_2-CH(CF_3)-CH_2-CF_3$ | 2 | H | $CH_3$ | $CH_3$ | CH | O | |
| 2.192 | $-CH_2-CH(CF_3)-CH_2-CF_3$ | 2 | H | $OCH_3$ | Cl | CH | O | |
| 2.193 | $-CH_2-CH(CF_3)-CH_2-CF_3$ | 2 | H | $OCH_3$ | $-N(CH_3)_2$ | CH | O | |
| 2.194 | $-CH_2-CH(CF_3)-CH_2-CF_3$ | 2 | H | $OCH_3$ | $CH_2F$ | CH | O | |
| 2.195 | $-CH_2-CH(CF_3)-CH_2-CF_3$ | 2 | H | $OCH_3$ | $-CH-OCH_3$ | CH | O | |
| 2.196 | $-CH_2-CH(CF_3)-CH_2-CF_3$ | 2 | H | $OCH_3$ | $OCH_3$ | CH | O | |
| 2.197 | $-CHCl-CHCl-CF_3$ | 2 | H | $OCH_3$ | $CH_3$ | N | O | |

Formulierungsbeispiele

Beispiel F1 : Formulierungsbeispiele für Wirkstoffe der Formel I (% = Gewichtsprozent

| a) *Spritzpulver* | a) | b) | c) |
|---|---|---|---|
| Wirkstoff | 20 % | 60 % | 0,5 % |
| Na-Ligninsulfonat | 5 % | 5 % | 5 % |
| Na-Laurylsulfat | 3 % | — | — |
| Na-Diisobutylnaphthalinsulfonat | — | 6 % | 6 % |
| Octylphenolpolyäthylenglykoläther (7-8 Mo AeO) | — | 2 % | 2 % |
| Hochdisperse Kieselsäure | 5 % | 27 % | 27 % |
| Kaolin | 67 % | — | — |
| Natriumchlorid | — | — | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) *Emulsions-Konzentrat* | a) | b) |
|---|---|---|
| Wirkstoff | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) *Stäubemittel* | a) | b) |
|---|---|---|
| Wirkstoff | 0,1 % | 1 % |
| Talkum | 99,9 % | — |
| Kaolin | — | 99 % |

Man erhält anwendungsfertige Stäubemittel, indem der Wirkstoff mit dem Träger vermischt und auf einer geeigneten Mühle vermahlen wird.

| d) *Extruder-Granulat* | a) | b) |
|---|---|---|
| Wirkstoff | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) *Umhüllungsgranulat* | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) *Suspensions-Konzentrat* | a) | b) |
|---|---|---|
| Wirkstoff | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37%ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein

Suspensions-Konzentrat, aus welchem durch Verdünnen mit Wasser Suspensionen jeder gewünschten Konzentration hergestellt werden können.

g) *Salzlösung*

| | |
|---|---|
| Wirkstoff | 5 % |
| Isopropylamin | 1 % |
| Octylphenolpolyäthylenglykoläther (78 Mol AeO) | 3 % |
| Wasser | 91 % |

## Biologische Beispiele

### Beispiel B1 : Herbizidwirkung vor dem Auflaufen der Pflanzen

Kunststofftöpfe werden mit expandiertem Vermiculit (Dichte : 0,135 g/$cm^3$, Wasserabsorptionsvermögen : 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät : Nasturtium officinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während der Keimphase von 4 bis 5 Tagen werden die Töpfe zur Erhöhung der örtlichen Luftfeuchtigkeit mit lichtdurchlässigem Material abgedeckt und mit deionisiertem Wasser gegossen. Nach dem 5. Tag wird dem Giesswasser 0,5 % eines handelsüblichen Flüssigdüngers (® Greenzit, ex Ciba-Geigy) zugesetzt. 12 Tage nach der Aussaat wird der Versuch ausgewertet und die Wirkung auf die Versuchspflanzen nach dem folgenden Massstab bewertet :

1 : Pflanze nicht gekeimt oder total abgestorben
2-3 : sehr starke Wirkung
4-6 : mittlere Wirkung
7-8 : schwache Wirkung
9 : keine Wirkung (wie unbehandelte Kontrolle).

pre-emergente Wirkung

Konzentration der Wirkstoffemulsion : 70,8 ppm

| Testpflanze / Wirkstoff Nr. | Nasturtium | Stellaria | Agrostis | Digitaria |
|---|---|---|---|---|
| 2.1 | 4 | 7 | 7 | 9 |
| 2.8 | 4 | 4 | 6 | 9 |
| 2.56 | 2 | 2 | 2 | 7 |
| 2.57 | 2 | 6 | 3 | 7 |
| 2.58 | 2 | 2 | 3 | 4 |
| 2.69 | 3 | 2 | 3 | 4 |
| 2.98 | 1 | 2 | 1 | 2 |
| 2.101 | 1 | 3 | 1 | 5 |
| 2.102 | 1 | 2 | 1 | 3 |
| 2.104 | 1 | 1 | 1 | 1 |

### Beispiel B2 : Nachweis der selektiven Herbizidwirkung vor dem Auflaufen der Pflanzen

Im Gewächshaus werden eine Anzahl Pflanzensamen in Blumentöpfe von 12-15 cm Durchmesser gesät. Unmittelbar danach wird die Erdoberfläche mit einer wässrigen Dispersion oder Lösung der Wirkstoffe behandelt. Es werden Konzentrationen von 500, 125 und 30 g Wirkstoffmengen pro Hektar angewendet. Die Töpfe werden dann im Gewächshaus bei einer Temperatur von 22-25 °C und 50-70 % relativer Luftfeuchtigkeit gehalten. Nach 3 Wochen wird der Versuch ausgewertet und die Wirkung nach dem in Beispiel B1 angegebenen Massstab beurteilt.

(Siehe Tabelle Seite 18 f.)

| Wirkung / Aufwandmenge g AS/ha / Testpflanze | Verb. Nr. 2.56 500 | 125 | 30 |
|---|---|---|---|
| Gerste | 7 | 8 | 9 |
| Weizen | 8 | 9 | 9 |
| Mais | 6 | 7 | 9 |
| Alopecurus myos. | 2 | 4 | 5 |
| Cyperus escul. | 2 | 4 | 6 |
| Abutilon | 1 | 2 | 3 |
| Sida spinosa | 2 | 2 | 3 |
| Xanthium Sp. | 2 | 2 | 2 |
| Amaranthus ret. | 1 | 2 | 2 |
| Chenopodium Sp. | 2 | 2 | 3 |
| Solanum nigrum | 3 | 3 | 3 |
| Ipomoea | 2 | 2 | 2 |
| Sinapis | 2 | 2 | 2 |
| Stellaria | 2 | 2 | 2 |
| Chrysanthe. leuc. | 2 | 2 | 2 |
| Viola tricolor | 2 | 2 | 2 |
| Veronica Sp. | 1 | 1 | 2 |

Beispiel B3 : Wuchshemmung bei tropischen Bodenbedecker-Leguminosen (cover crops)

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6 000 lux Kunstlicht, pro Tag 14 Stunden, bei Temperaturen von 27° bei Tag und 21 °C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Formel I behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel B4 : Wuchsregulierung an Sojabohnen

In Kunststoffbehältern mit einem Erde-Torf-Sandgemisch im Verhältnis 6 : 3 : 1 werden Sojabohnen der Sorte « Hark » angesät und in eine Klimakammer gegeben. Durch optimale Temperaturwahl, Beleuchtung, Düngerzugabe und Bewässerung entwickln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Formel I eine merkliche Erhöhung der Anzahl und des Gewichts der Schoten im Haupttrieb.

Beispiel B5 : Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Formel I besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel B6 : Wuchshemmung bei Gräsern

In Kunststoffschalen mit Erde-Torf-Sand-Gemisch (6 : 3 : 1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchtentlich bis auf 4 cm Höhe zurückgeschnitten und ca. 50 Tage nach der Aussaat und einen Tag nach dem letzten Schnitt mit der wässrigen Spritzbrühe eines Wirkstoffes der Formel I besprüht. Die Wirkstoffmenge beträgt umgerechnet bis zu 100 g Aktivsubstanz pro Hektar. 21 Tage nach Applikation wird das Wachstum der Gräser beurteilt.

Die Verbindungen der Formel I bewirken eine Reduzierung des Neuzuwachses von um 10-30 % im Vergleich zur unbehandelten Kontrolle.

**Patentansprüche** (für die Vertragsstaaten : BE, DE, FR, IT, NL, CH, LI, GB)

1. N-Phenylsulfonyl-N'-pyrimidinyl- und -triazinyl-harnstoffe der Formel I

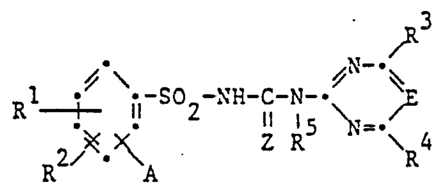

(I)

worin

A $C_1$-$C_6$-Halogenalkyl,

$R^1$ Wasserstoff, Halogen, Nitro, Cyan, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, —CO—$R^6$, —$NR^7R^8$, —CO—$NR^9R^{10}$ oder —$SO_2$—$NR^{11}R^{12}$,

$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,

$R^3$ und $R^4$ unabhängig voneinander Wasserstoff, Halogen, $C_1$-$C_4$-Alkyl, $C_1$-$C_4$-Halogenalkyl, $C_2$-$C_4$-Halogenalkoxy, $C_1$-$C_4$-Alkylthio, $C_1$-$C_4$-Halogenalkylthio, $C_2$-$C_4$-Alkoxyalkyl, Methoxy, Aethoxy oder —$NR^{12}R^{13}$,

$R^5$ Wasserstoff, $C_1$-$C_4$-Alkyl oder $C_1$-$C_4$-Alkoxy,

$R^6$ $C_1$-$C_4$-Alkoxy, $C_1$-$C_4$-Halogenalkoxy, $C_1$-$C_4$-Alkylthio, $C_2$-$C_6$-Alkoxyalkoxy, Wasserstoff, $C_1$-$C_4$-Alkyl oder $C_1$-$C_4$-Halogenalkyl,

$R^7$, $R^8$, $R^9$, $R^{10}$, $R^{11}$, $R^{12}$ und $R^{13}$ unabhängig voneinander Wasserstoff oder $C_1$-$C_4$-Alkyl,

E Stickstoff oder die Methinbrücke und

Z Sauerstoff oder Schwefel bedeuten,

sowie die Salze dieser Verbindungen, mit der Massgabe, dass A nicht für Trifluormethyl, 2-Fluoräthyl, 2-Chloräthyl, 2-Bromäthyl, 2-Chlor-2-fluoräthyl, 2,2-Dichloräthyl, 2-Brom-2-chloräthyl, 2-Brom-2-fluoräthyl, 2,2-Dibromäthyl, 2-Fluorpropyl, 2-Chlorpropyl, 2-Brompropyl oder eine Gruppe —$CR^aR^bR^c$ steht, wobei $R^a$ Wasserstoff, Chlor oder $C_1$-$C_4$-Alkyl, $R^b$ Wasserstoff oder Methyl und $R^c$ Chlor oder Brom bedeuten.

2. Verbindungen gemäss Anspruch 1, dadurch gekennzeichnet, dass $R^1$ Wasserstoff ist.

3. Verbindungen gemäss Anspruch 1, dadurch gekennzeichnet, dass $R^2$ Wasserstoff ist.

4. Verbindungen gemäss Anspruch 1, dadurch gekennzeichnet, dass Z für Sauerstoff steht.

5. Verbindungen gemäss Anspruch 1, dadurch gekennzeichnet, dass $R^5$ Wasserstoff ist.

6. Verbindungen gemäss Anspruch 1, dadurch gekennzeichnet, dass $R^3$ und $R^4$ zusammen höchstens 4 Kohlenstoffatome enthalten.

7. Verbindungen gemäss Anspruch 1, dadurch gekennzeichnet, dass der Rest A mindestens ein Fluoratom enthält.

8. Verbindungen gemäss Anspruch 1, dadurch gekennzeichnet, dass der Rest A die 2-Stellung des Phenylrings besetzt.

9. Verbindungen gemäss Anspruch 1, dadurch gekennzeichnet, dass $R^1$, $R^2$ und $R^5$ für Wasserstoff und Z für Sauerstoff stehen.

10. Verbindungen gemäss Anspruch 1, dadurch gekennzeichnet, dass $R^3$ und $R^4$ zusammen höchstens 4 Kohlenstoffatome enthalten, der Rest A die 2-Stellung des Phenylrings besetzt und mindestens ein Fluoratom enthält.

11. Verbindungen gemäss Anspruch 1, dadurch gekennzeichnet, dass $R^1$, $R^2$ und $R^5$ für Wasserstoff und Z für Sauerstoff stehen, $R^3$ und $R^4$ zusammen höchstens 4 Kohlenstoffatome enthalten und der Rest A die 2-Stellung des Phenylrings besetzt und mindestens ein Fluoratom enthält.

12. Verbindungen gemäss Anspruch 11, dadurch gekennzeichnet, dass A für einen Rest aus der Gruppe 3,3,3-Trifluorpropyl, Fluormethyl, Difluormethyl, 3,3,4,4,5,5,5-Heptafluorpentyl, 4,4,4-Trifluor-2-trifluormethyl-butyl, Chlorfluormethyl, Bromfluormethyl, 3,3-Difluorbutyl oder 3-Fluorpropyl steht.

13. N-[2-(3,3,3-Trifluorpropyl)-phenyl-sulfonyl]-N'-(4,6-dimethoxypyrimidin-2-yl)-harnstoff gemäss Anspruch 1.

14. N-[2-(3,3,3-Trifluorpropyl)-phenylsulfonyl]-N'-(4,6-dimethoxy-1,3,5-triazin-2-yl)-harnstoff gemäss Anspruch 1.

15. N-[2-(3,3-Difluorbutyl)-phenylsulfonyl]-N'-(4-methoxy-6-methyl-1,3,5-triazin-2-yl)-harnstoff gemäss Anspruch 1.

16. N-[2-(3,3,3-Trifluorpropyl)-phenylsulfonyl]-N'-(4-methoxy-6-methyl-1,3,5-triazin-2-yl)-harnstoff gemäss Anspruch 1.

17. Phenylsulfonamide der Formel II

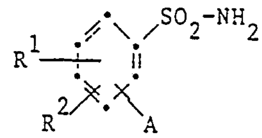

(II)

worin $R^1$, $R^2$ und A die unter Formel I im Anspruch 1 gegebene Bedeutung haben.

18. Phenylsulfonylisocyanate und -isothiocyanate der Formel IV

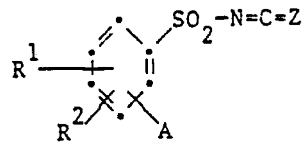

(IV)

worin $R^1$, $R^2$, A und Z die unter Formel I in Anspruch 1 gegebene Bedeutung haben.

19. N-Phenylsulfonylcarbamate der Formel VII

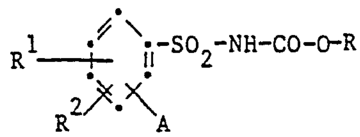

(VII)

worin $R^1$, $R^2$ und A die unter Formel I in Anspruch 1 gegebene Bedeutung haben und R für Phenyl, Alkyl oder substituiertes Phenyl steht.

20. Verfahren zur Herstellung der Verbindungen der Formel I, dadurch gekennzeichnet, dass man ein Phenylsulfonamid der Formel II

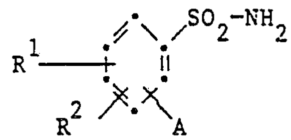

(II)

worin A, $R^1$ und $R^2$ die unter Formel I gegebene Bedeutung haben, in Gegenwart einer Base mit einem N-Pyrimidinyl- oder -Triazinylcarbamat der Formel III

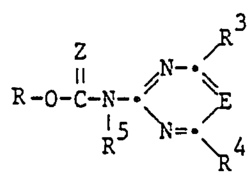

(III)

worin E, $R^3$, $R^4$, $R^5$ und Z die unter Formel I gegebene Bedeutung haben und R für Phenyl, Alkyl oder substituiertes Phenyl steht, umsetzt und gegebenenfalls in ihre Salze überführt.

21. Verfahren zur Herstellung der Verbindungen der Formel I, dadurch gekennzeichnet, dass man ein Phenylsulfonylisocyanat oder -isothiocyanat der Formel IV

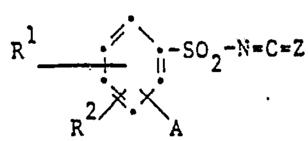

(IV)

worin A, $R^1$, $R^2$ und Z die unter Formel I gegebene Bedeutung haben, gegebenenfalls in Gegenwart einer Base, mit einem Amin der Formel V

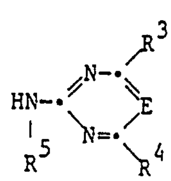

(V)

worin E, $R^3$, $R^4$ und $R^5$ die unter Formel I gegebene Bedeutung haben, umsetzt und gegebenenfalls in ihre Salze überführt.

22. Verfahren zur Herstellung der Verbindungen der Formel I in denen $R^5$ Wasserstoff ist, dadurch gekennzeichnet, dass man ein Sulfonamid der Formel II gemäss Anspruch 20 gegebenenfalls in Gegenwart einer Base mit einem Isocyanat oder Isothiocyanat der Formel VI

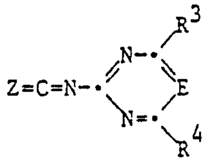

(VI)

worin E, $R^3$, $R^4$, $R^5$ und Z die unter Formel I gegebene Bedeutung haben, umsetzt und gegebenenfalls in ihre Salze überführt.

23. Verfahren zur Herstellung der Verbindungen der Formel I, dadurch gekennzeichnet, dass man ein N-Phenylsulfonylcarbamat der Formel VII

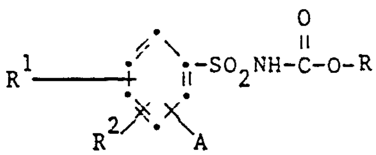

(VII)

worin A, $R^1$ und $R^2$ die unter Formel I gegebene Bedeutung haben und R für Phenyl, Alkyl oder substituiertes Phenyl steht, mit einem Amin der Formel V gemäss Anspruch 21 umsetzt und gegebenenfalls in ihre Salze überführt.

24. Verfahren zur Herstellung der Verbindungen der Formel I, in denen A $C_2$-$C_6$-Halogenalkyl bedeutet, dadurch gekennzeichnet, dass man einen Sulfonylharnstoff der Formel VIII

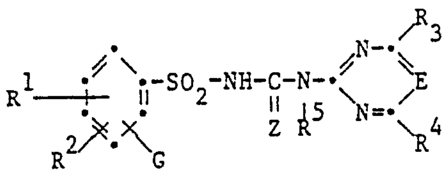

(VIII)

worin $R^1$, $R^2$, $R^3$, $R^4$, $R^5$, E und Z die unter Formel I gegebene Bedeutung haben und G für einen ungesättigen $C_2$-$C_6$-Halogenalkenylrest steht, bis zur Sättigung hydriert oder halogeniert und gegebenenfalls in ihre Salze überführt.

25. Verfahren zur Herstellung der Verbindungen der Formel I, in denen A $C_2$-$C_6$-Halogenalkyl bedeutet, dadurch gekennzeichnet, dass man einen Sulfonylharnstoff der Formel IX

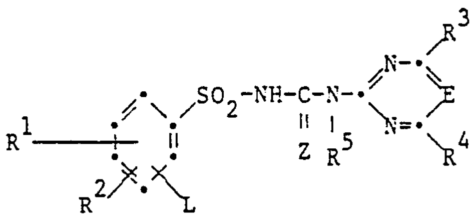

(IX)

worin $R^1$, $R^2$, $R^3$, $R^4$, $R^5$, E und Z die unter Formel I gegebene Bedeutung haben und L für einen ungesättigten $C_2$-$C_6$-Alkylrest stehen, bis zur Sättigung halogeniert und gegebenenfalls in ihre Salze überführt.

26. Verfahren zur Herstellung von Additionssalzen der Formel I gemäss einem der Ansprüche 20 bis 25, dadurch gekennzeichnet, dass man einen Sulfonylharnstoff der Formel I mit einem Amin, einem Alkalimetall- oder Erdalkalimetallhydroxid oder einer quaternären Ammoniumbase umsetzt.

27. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder pyrimidinyl-harnstoff der Formel I, Anspruch 1, enthält.

28. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

29. Die Verwendung der N-Phenylsulfonyl-N'-triazinyl- oder -pyrimidinyl-harnstoffe der Formel I, Anspruch 1, oder sie enthaltender Mittel zur Regulierung des Pflanzenwachstums.

30. Die Verwendung der N-Phenylsulfonyl-N'-triazinyl- oder -pyrimidinyl-harnstoffe der Formel I, oder sie enthaltender Mittel, gemäss Anspruch 28, zur selektiven pre- oder postemergenten Bekämpfung von Unkräutern in Nutzpflanzenkulturen.

31. Die Verwendung gemäss Anspruch 30 in Zuckerrohr-, Getreide-, Mais- und Baumwollkulturen.

32. Die Verwendung gemäss Anspruch 30 in Sojakulturen.

33. Die Verwendung gemäss Anspruch 30 in Reiskulturen.
34. Die Verwendung gemäss Anspruch 29 bei Bodendecker-Leguminosen.
35. Phenylsulfonylchloride der Formel

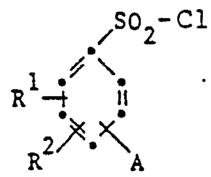

worin $R^1$, $R^2$ und A die unter Formel I im Anspruch 1 gegebene Bedeutung haben.

**Patentansprüche** (für den Vertragsstaat AT)

1. Ein herbizides und den Pflanzenwuchs regulierendes Mittel, dadurch gekennzeichnet, dass es neben Träger- und/oder anderen Zuschlagstoffen als Wirkstoff mindestens einen N-Phenylsulfonyl-N'-triazinyl- oder pyrimidinyl-harnstoff der Formel I

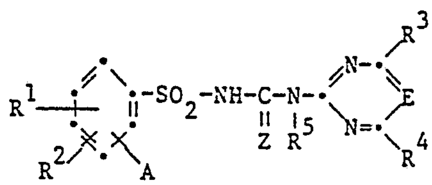

(I)

oder ein Salz davon enthält, worin
A $C_1$-$C_6$-Halogenalkyl,
$R^1$ Wasserstoff, Halogen, Nitro, Cyan, $C_1$-$C_4$-Alkyl, $C_1$-$C_4$-Halogenalkyl, $C_1$-$C_4$-Alkoxy, $C_1$-$C_4$-Alkylthio, $C_1$-$C_4$-Alkylsulfinyl, $C_1$-$C_4$-Alkylsulfonyl, —CO-$R^6$, —$NR^7R^8$, —CO—$NR^9R^{10}$ oder —$SO_2$—$NR^{11}R^{12}$,
$R^2$ Wasserstoff, Halogen, $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl,
$R^3$ und $R^4$ unabhängig voneinander Wasserstoff, Halogen, $C_1$-$C_4$-Alkyl, $C_1$-$C_4$-Halogenalkyl, $C_2$-$C_4$-Halogenalkoxy, $C_1$-$C_4$-Alkylthio, $C_1$-$C_4$-Halogenalkylthio, $C_2$-$C_4$-Alkoxyalkyl, Methoxy, Aethoxy oder —$NR^{12}R^{13}$,
$R^5$ Wasserstoff, $C_1$-$C_4$-Alkyl oder $C_1$-$C_4$-Alkoxy,
$R^6$ $C_1$-$C_4$-Alkoxy, $C_1$-$C_4$-Halogenalkoxy, $C_1$-$C_4$-Alkylthio, $C_2$-$C_6$-Alkoxyalkoxy, Wasserstoff, $C_1$-$C_4$-Alkyl oder $C_1$-$C_4$-Halogenalkyl,
$R^7$, $R^8$, $R^9$, $R^{10}$, $R^{11}$, $R^{12}$ und $R^{13}$ unabhängig voneinander Wasserstoff oder $C_1$-$C_4$-Alkyl,
E Stickstoff oder die Methinbrücke und
Z Sauerstoff oder Schwefel bedeuten,
mit der Massgabe, dass A nicht für Trifluormethyl, 2-Fluoräthyl, 2-Chloräthyl, 2-Bromäthyl, 2-Chlor-2-fluoräthyl, 2,2-Dichloräthyl, 2-Brom-2-chloräthyl, 2-Brom-2-fluoräthyl, 2,2-Dibromäthyl, 2-Fluorpropyl, 2-Chlorpropyl, 2-Brompropyl oder eine Gruppe —$CR^aR^bR^c$ steht, wobei $R^a$ Wasserstoff, Chlor oder $C_1$-$C_4$-Alkyl, $R^b$ Wasserstoff oder Methyl und $R^c$ Chlor oder Brom bedeuten.

2. Mittel gemäss Anspruch 1, dadurch gekennzeichnet, dass $R^1$ Wasserstoff ist.
3. Mittel gemäss Anspruch 1, dadurch gekennzeichnet, dass $R^2$ Wasserstoff ist.
4. Mittel gemäss Anspruch 1, dadurch gekennzeichnet, dass Z für Sauerstoff steht.
5. Mittel gemäss Anspruch 1, dadurch gekennzeichnet, dass $R^5$ Wasserstoff ist.
6. Mittel gemäss Anspruch 1, dadurch gekennzeichnet, dass $R^3$ und $R^4$ zusammen höchstens 4 Kohlenstoffatome enthalten.
7. Mittel gemäss Anspruch 1, dadurch gekennzeichnet, dass der Rest A mindestens ein Fluoratom enthält.
8. Mittel gemäss Anspruch 1, dadurch gekennzeichnet, dass der Rest A die 2-Stellung des Phenylrings besetzt.
9. Mittel gemäss Anspruch 1, dadurch gekennzeichnet, dass $R^1$, $R^2$ und $R^5$ für Wasserstoff und Z für Sauerstoff stehen.
10. Mittel gemäss Anspruch 1, dadurch gekennzeichnet, dass $R^3$ und $R^4$ zusammen höchstens 4 Kohlenstoffatome enthalten, der Rest A die 2-Stellung des Phenylrings besetzt und mindestens ein Fluoratom enthält.
11. Mittel gemäss Anspruch 1, dadurch gekennzeichnet, dass $R^1$, $R^2$ und $R^5$ für Wasserstoff und Z für Sauerstoff stehen, $R^3$ und $R^4$ zusammen höchstens 4 Kohlenstoffatome enthalten und der Rest A die 2-Stellung des Phenylrings besetzt und mindestens ein Fluoratom enthält.
12. Mittel gemäss Anspruch 11, dadurch gekennzeichnet, dass A für einen Rest aus der Gruppe 3,3,3-Trifluorpropyl, Fluormethyl, Difluormethyl, 3,3,4,4,5,5,5-Heptafluorpentyl, 4,4,4-Trifluor-2-trifluormethylbutyl, Chlorfluormethyl, Bromfluormethyl, 3,3-Difluorbutyl oder 3-Fluorpropyl steht.

13. Mittel gemäss Anspruch 1, dadurch gekennzeichnet, dass es als Wirkstoff N-[2-(3,3,3-Trifluorpropyl)-phenyl-sulfonyl]-N'-(4,6-dimethoxypyrimidin-2-yl)-harnstoff enthält.

14. Mittel gemäss Anspruch 1, dadurch gekennzeichnet, dass es als Wirkstoff N-[2-(3,3,3-Trifluorpropyl)-phenylsulfonyl]-N'-(4,6-dimethoxy-1,3,5-triazin-2-yl)-harnstoff enthält.

15. Mittel gemäss Anspruch 1, dadurch gekennzeichnet, dass es als Wirkstoff N-[2-(3,3-Difluorbutyl)-phenylsulfonyl]-N'-(4-methoxy-6-methyl-1,3,5-triazin-2-yl)-harnstoff enthält.

16. Mittel gemäss Anspruch 1, dadurch gekennzeichnet, dass es als Wirkstoff N-[2-(3,3,3-Trifluorpropyl)-phenylsulfonyl]-N'-(4-methoxy-6-methyl-1,3,5-triazin-2-yl)-harnstoff enthält.

17. Verfahren zur Herstellung der Verbindungen der Formel I, dadurch gekennzeichnet, dass man entweder

a) ein Phenylsulfonamid der Formel II

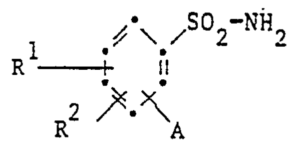

(II)

worin A, $R^1$ und $R^2$ die unter Formel I gegebene Bedeutung haben, in Gegenwart einer Base mit einem N-Pyrimidinyl- oder -Triazinylcarbamat der Formel III

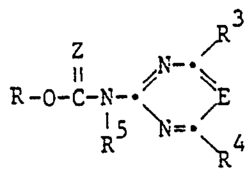

(III)

worin E, $R^3$, $R^4$, $R^5$ und Z die unter Formel I gegebene Bedeutung haben und R für Phenyl, Alkyl oder substituiertes Phenyl steht, umsetzt oder

b) ein Phenylsulfonylisocyanat oder -isothiocyanat der Formel IV

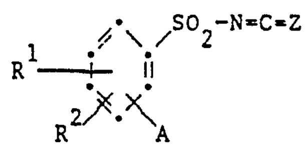

(IV)

worin A, $R^1$, $R^2$ und Z die unter Formel I gegebene Bedeutung haben, gegebenenfalls in Gegenwart einer Base, mit einem Amin der Formel V

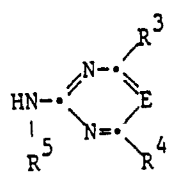

(V)

worin E, $R^3$, $R^4$ und $R^5$ die unter Formel I gegebene Bedeutung haben, umsetzt oder

c) ein N-Phenylsulfonylcarbamat der Formel VII

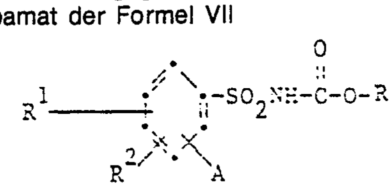

(VII)

worin A, $R^1$ und $R^2$ die unter Formel I gegebene Bedeutung haben und R für Phenyl, Alkyl oder substituiertes Phenyl steht, mit einem Amin der Formel V umsetzt und gegebenenfalls in ihre Salze überführt indem man einen Sulfonylharnstoff der Formel I mit einem Amin, einem Alkalimetall- oder Erdalkalimetallhydroxid oder einer quaternären Ammoniumbase umsetzt.

18. Die Verwendung der N-Phenylsulfonyl-N'-triazinyl- oder -pyrimidinyl-harnstoffe der Formel I, nach Anspruch 1, oder sie enthaltender Mittel zur Bekämpfung unerwünschten Pflanzenwachstums.

19. Die Verwendung der N-Phenylsulfonyl-N'-triazinyl- oder -pyrimidinyl-harnstoffe der Formel I, nach Anspruch 1, oder sie enthaltender Mittel zur Regulierung des Pflanzenwachstums.

20. Die Verwendung gemäss Anspruch 18, zur selektiven pre- oder postemergenten Bekämpfung von Unkräutern in Nutzpflanzenkulturen.

21. Die Verwendung gemäss Anspruch 20 in Zuckerrohr-, Getreide-, Mais- und Baumwollkulturen.
22. Die Verwendung gemäss Anspruch 20 in Sojakulturen.
23. Die Verwendung gemäss Anspruch 20 in Reiskulturen.
24. Die Verwendung gemäss Anspruch 19 bei Bodendecker-Leguminosen.

**Claims** (for the Contracting States : BE, DE, FR, IT, NL, CH, LI, GB)

1. N-phenylsulfonyl-N'-pyrimidinyl- and -triazinyl-ureas of the formula I

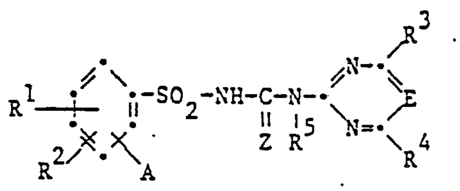

(I)

wherein

A is $C_1$-$C_6$-haloalkyl,

$R^1$ is hydrogen, halogen, nitro, cyano, $C_1$-$C_4$-alkyl, $C_1$-$C_4$-haloalkyl, $C_1$-$C_4$-alkoxy, $C_1$-$C_4$-alkylthio, $C_1$-$C_4$-alkylsulfinyl, $C_1$-$C_4$-alkylsulfonyl, —CO-$R^6$, —$NR^7R^8$, —CO—$NR^9R^{10}$ or —$SO_2$—$NR^{11}R^{12}$,

$R^2$ is hydrogen, halogen, $C_1$-$C_4$-alkyl, $C_1$-$C_4$-alkoxy, $C_1$-$C_4$-alkylthio, $C_1$-$C_4$-alkylsulfinyl or $C_1$-$C_4$-alkylsulfonyl,

$R^3$ und $R^4$ independently of one another are each hydrogen, halogen, $C_1$-$C_4$-alkyl, $C_1$-$C_4$-haloalkyl, $C_2$-$C_4$-haloalkoxy, $C_1$-$C_4$-alkylthio, $C_1$-$C_4$-haloalkylthio, $C_2$-$C_4$-alkoxyalkyl, methoxy, ethoxy or —$NR^{12}R^{13}$,

$R^5$ is hydrogen, $C_1$-$C_4$-alkyl or $C_1$-$C_4$-alkoxy,

$R^6$ is $C_1$-$C_4$-alkoxy, $C_1$-$C_4$-haloalkoxy, $C_1$-$C_4$-alkylthio, $C_2$-$C_6$-alkoxyalkoxy, hydrogen, $C_1$-$C_4$-alkyl or $C_1$-$C_4$-haloalkyl,

$R^7$, $R^8$, $R^9$, $R^{10}$, $R^{11}$, $R^{12}$ and $R^{13}$ independently of one another are each hydrogen or $C_1$-$C_4$-alkyl,

E is nitrogen or the methine bridge, and

Z is oxygen or sulfur,

and also the salts of these compounds, with the proviso that A is not trifluoromethyl, 2-fluoroethyl, 2-chloroethyl, 2-bromoethyl, 2-chloro-2-fluoroethyl, 2,2-dichloroethyl, 2-bromo-2-chloroethyl, 2-bromo-2-fluoroethyl, 2,2-dibromoethyl, 2-fluoropropyl, 2-chloropropyl, 2-bromopropyl, or a group —$CR^aR^bR^c$, where $R^a$ is hydrogen, chlorine or $C_1$-$C_4$-alkyl, $R^b$ is hydrogen or methyl, and $R^c$ is chlorine or bromine.

2. Compounds according to claim 1, characterised in that $R^1$ is hydrogen.
3. Compounds according to claim 1, characterised in that $R^2$ is hydrogen.
4. Compounds according to claim 1, characterised in that Z is oxygen.
5. Compounds according to claim 1, characterised in that $R^5$ is hydrogen.
6. Compounds according to claim 1, characterised in that $R^3$ and $R^4$ together contain at most 4 carbon atoms.
7. Compounds according to claim 1, characterised in that the radical A contains at least one fluorine atom.
8. Compounds according to claim 1, characterised in that the radical A occupies the 2-position of the phenyl ring.
9. Compounds according to claim 1, characterised in that $R^1$, $R^2$ and $R^5$ are hydrogen and Z is oxygen.
10. Compounds according to claim 1, characterised in that $R^3$ and $R^4$ together contain at most 4 carbon atoms, and the radical A occupies the 2-position of the phenyl ring and contains at least one fluorine atom.
11. Compounds according to claim 1, characterised in that $R^1$ and $R^2$ and $R^5$ are hydrogen and Z is oxygen, $R^3$ and $R^4$ together contain at most 4 carbon atoms, and the radical A occupies the 2-position of the phenyl ring and contains at least one fluorine atom.
12. Compounds according to claim 11, characterised in that A is a radical from the group comprising : 3,3,3-trifluoropropyl, fluoromethyl, difluoromethyl, 3,3,4,4,5,5,5-heptafluoropentyl, 4,4,4-trifluoro-2-trifluoromethylbutyl, chlorofluoromethyl, bromofluoromethyl, 3,3-difluorobutyl or 3-fluoropropyl.
13. N-[2-(3,3,3-Trifluoropropyl)-phenylsulfonyl]-N'-(4,6-dimethoxy-pyrimidin-2-yl)-urea according to claim 1.
14. N-[2-(3,3,3-Trifluoropropyl)-phenylsulfonyl]-N'-(4,6-dimethoxy-1,3,5-triazin-2-yl)-urea according to claim 1.
15. N-[2-(3,3,-Difluorobutyl)-phenylsulfonyl]-N'-(4-methoxy-6-methyl-1,3,5-triazin-2-yl)-urea according to claim 1.
16. N-[2-(3,3,3-Trifluoropropyl)-phenylsulfonyl]-N'-(4-methoxy-6-methyl-1,3,5-triazin-2-yl)-urea according to claim 1.

17. Phenylsulfonamides of the formula II

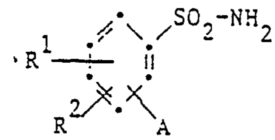

(II)

wherein $R^1$, $R^2$ and A have the meanings defined under the formula I in claim 1.

18. Phenylsulfonylisocyanates and -isothiocyanates of the formula IV

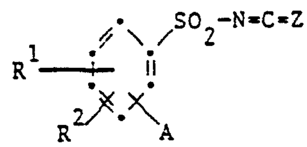

(IV)

wherein $R^1$, $R^2$, A and Z have the meanings defined under the formula I in claim 1.

19. N-phenylsulfonylcarbamates of the formula VII

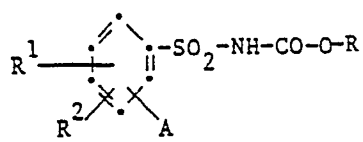

(VII)

wherein $R^1$, $R^2$ and A have the meanings defined under the formula I in claim 1, and R is phenyl, alkyl or substituted phenyl.

20. A process for producing the compounds of the formula I, characterised in that a phenylsulfonamide of the formula II

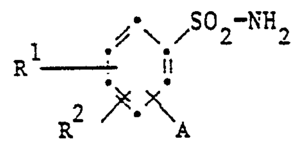

(II)

wherein A, $R^1$ and $R^2$ have the meanings defined under the formula I, is reacted in the presence of a base with an N-pyrimidinyl- or -triazinylcarbamate of the formula III

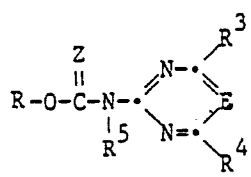

(III)

wherein E, $R^3$, $R^4$, $R^5$ and Z have the meanings defined under the formula I, and R is phenyl, alkyl or substituted phenyl, and the compounds obtained are optionally converted into salts thereof.

21. A process for producing the compounds of the formula I, characterised in that a phenylsulfonylisocyanate or -isothiocyanate of the formula IV

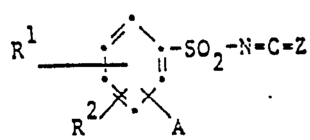

(IV)

wherein A, $R^1$, $R^2$ and Z have the meanings defined under the formula I, is reacted, optionally in the presence of a base, with an amine of the formula V

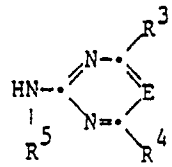

(V)

wherein E, $R^3$, $R^4$ and $R^5$ have the meanings defined under the formula I, and the compounds obtained are optionally converted into salts thereof.

22. A process for producing the compounds of the formula I in which $R^5$ is hydrogen, characterised in that a sulfonamide of the formula II according to claim 20 is reacted, optionally in the presence of a base, with an isocyanate or isothiocyanate of the formula VI

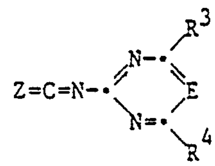

(VI)

wherein E, $R^3$, $R^4$, $R^5$ and Z have the meanings defined under the formula I, and the compounds obtained are optionally converted into salts thereof.

23. A process for producing the compounds of the formula I, characterised in that an N-phenylsulfonylcarbamate of the formula VII

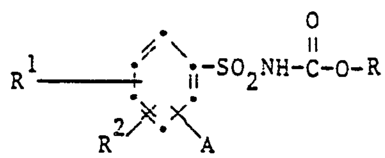

(VII)

wherein A, $R^1$ and $R^2$ have the meanings defined under the formula I, and R is phenyl, alkyl or substituted phenyl, is reacted with an amine of the formula V according to claim 21, and the products obtained are optionally converted into salts thereof.

24. A process for producing the compounds of the formula I in which A is $C_2$-$C_6$-haloalkyl, characterised in that a sulfonylurea of the formula VIII

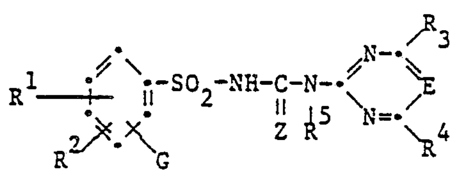

(VIII)

wherein $R^1$, $R^2$, $R^3$, $R^4$, $R^5$, E and Z have the meanings defined under the formula I, and G is an unsaturated $C_2$-$C_6$-haloalkenyl radical, is hydrogenated or halogenated to saturation and the compounds obtained are optionally converted into salts thereof.

25. A process for producing the compounds of the formula I in which A is $C_2$-$C_6$-haloalkyl, characterised in that a sulfonylurea of the formula IX

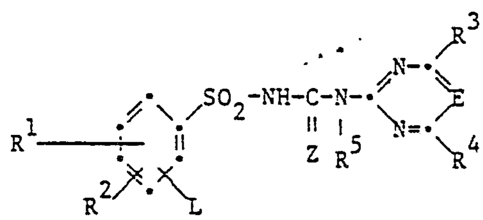

(IX)

wherein $R^1$, $R^2$, $R^3$, $R^4$, $R^5$, E and Z have the meanings defined under the formula I, and L is an unsaturated $C_2$-$C_6$-alkyl radical, is halogenated to saturation and the compounds obtained are optionally converted into salts thereof.

26. A process for producing addition salts of the formula I according to any one of claims 20 to 25, characterised in that a sulfonylurea of the formula I is reacted with an amine, with an alkali metal hydroxide or alkaline earth metal hydroxide or with a quaternary ammonium base.

27. A herbicidal and plant-growth-regulating composition, characterised in that it contains, as active ingredient, at least one N-phenylsulfonyl-N'-triazinyl- or -pyrimidinylurea of the formula I, claim 1, together with carriers and/or other additives.

28. The use of the N-phenylsulfonyl-N'-triazinyl- or -pyrimidinylureas of the formula I, claim 1, or of compositions containing them for controlling undesired plant growth.

29. The use of the N-phenylsulfonyl-N'-triazinyl- or -pyrimidinylureas of the formula I, claim 1, or of compositions containing them for regulating plant growth.

30. The use of the N-phenylsulfonyl-N'-triazinyl- or pyrimidinylureas of the formula I, or of compositions containing them, according to claim 28, for the selective, pre- or post-emergence controlling of weeds in crops of useful plants.

31. The use according to claim 30 in crops of sugar cane, cereals, maize and cotton.

32. The use according to claim 30 in soya-bean crops.

33. The use according to claim 30 in rice crops.

34. The use according to claim 29 in leguminous cover crops.

35. Phenylsulfonyl chlorides of the formula

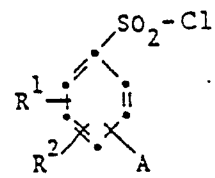

wherein $R^1$, $R^2$ and A have the meanings defined under the formula I in claim 1.

**Claims** (for the Contracting State AT)

1. A herbicidal and plant-growth-regulating composition, characterised in that it contains, as active ingredient, at least one N-phenylsulfonyl-N'-triazinyl- or -pyrimidinylurea of the formula I

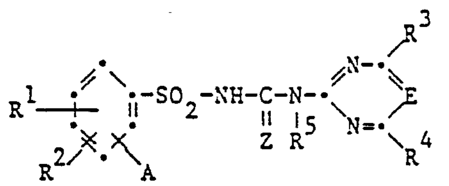

(I)

or a salt thereof, wherein

A is $C_1$-$C_6$-haloalkyl,

$R^1$ is hydrogen, halogen, nitro, cyano, $C_1$-$C_4$-alkyl, $C_1$-$C_4$-haloalkyl, $C_1$-$C_4$-alkoxy, $C_1$-$C_4$-alkylthio, $C_1$-$C_4$-alkylsulfinyl, $C_1$-$C_4$-alkylsulfonyl, -CO-$R^6$, -$NR^7R^8$, -CO-$NR^9R^{10}$ or -$SO_2$-$NR^{11}R^{12}$,

$R^2$ is hydrogen, halogen, $C_1$-$C_4$-alkyl, $C_1$-$C_4$-alkoxy, $C_1$-$C_4$-alkylthio, $C_1$-$C_4$-alkylsulfinyl or $C_1$-$C_4$-alkylsulfonyl,

$R^3$ and $R^4$ independently of one another are each hydrogen, halogen, $C_1$-$C_4$-alkyl, $C_1$-$C_4$-haloalkyl, $C_2$-$C_4$-haloalkoxy, $C_1$-$C_4$-alkylthio, $C_1$-$C_4$-haloalkylthio, $C_2$-$C_4$-alkoxyalkyl, methoxy, ethoxy or -$NR^{12}R^{13}$,

$R^5$ is hydrogen, $C_1$-$C_4$-alkyl or $C_1$-$C_4$-alkoxy,

$R^6$ is $C_1$-$C_4$-alkoxy, $C_1$-$C_4$-haloalkoxy, $C_1$-$C_4$-alkylthio, $C_2$-$C_6$-alkoxyalkoxy, hydrogen, $C_1$-$C_4$-alkyl or $C_1$-$C_4$-haloalkyl,

$R^7$, $R^8$, $R^9$, $R^{10}$, $R^{11}$, $R^{12}$ and $R^{13}$ independently of one another are each hydrogen or $C_1$-$C_4$-alkyl,

E is nitrogen or the methine bridge, and

Z is oxygen or sulfur,

with the proviso that A is not trifluoromethyl, 2-fluoroethyl, 2-chloroethyl, 2-bromoethyl, 2-chloro-2-fluoroethyl, 2,2-dichloroethyl, 2-bromo-2-chloroethyl, 2-bromo-2-fluoroethyl, 2,2-dibromoethyl, 2-fluoropropyl, 2-chloropropyl, 2-bromopropyl, or a group -$CR^aR^bR^c$, where $R^a$ is hydrogen, chlorine or $C_1$-$C_4$-alkyl, $R^b$ is hydrogen or methyl, and $R^c$ is chlorine or bromine, together with carriers and/or other additives.

2. A composition according to claim 1, characterised in that $R^1$ is hydrogen.

3. A composition according to claim 1, characterised in that $R^2$ is hydrogen.

4. A composition according to claim 1, characterised in that Z is oxygen.

5. A composition according to claim 1, characterised in that $R^5$ is hydrogen.

6. A composition according to claim 1, characterised in that $R^3$ and $R^4$ together contain at most 4 carbon atoms.

7. A composition according to claim 1, characterised in that the radical A contains at least one fluorine atom.

8. A composition according to claim 1, characterised in that the radical A occupies the 2-position of the phenyl ring.

9. A composition according to claim 1, characterised in that $R^1$, $R^2$ and $R^5$ are hydrogen and Z is oxygen.

10. A composition according to claim 1, characterised in that $R^3$ and $R^4$ together contain at most 4 carbon atoms, and the radical A occupies the 2-position of the phenyl ring and contains at least one fluorine atom.

11. A composition according to claim 1, characterised in that $R^1$, $R^2$ and $R^5$ are hydrogen and Z is oxygen, $R^3$ and $R^4$ together contain at most 4 carbon atoms, and the radical A occupies the 2-position of the phenyl ring and contains at least one fluorine atom.

12. A composition according to claim 11, characterised in that A is a radical from the group comprising : 3,3,3-trifluoropropyl, fluoromethyl, difluoromethyl, 3,3,4,4,5,5,5-heptafluoropentyl, 4,4,4-trifluoro-2-trifluoromethylbutyl, chlorofluoromethyl, bromofluoromethyl, 3,3-difluorobutyl or 3-fluoropropyl.

13. A composition according to claim 1, characterised in that it contains, as active ingredient, N-[2-(3,3,3-trifluoropropyl)-phenylsulfonyl]-N'-(4,6-dimethoxypyrimidin-2-yl)-urea.

14. A composition according to claim 1, characterised in that it contains, as active ingredient, N-[2-(3,3,3-trifluoropropyl)-phenylsulfonyl]-N'-(4,6-dimethoxy-1,3,5-triazin-2-yl)-urea.

15. A composition according to claim 1, characterised in that it contains, as active ingredient, N-[2-(3,3-difluorobutyl)-phenylsulfonyl]-N'-(4-methoxy-6-methyl-1,3,5-triazin-2-yl)-urea.

16. A composition according to claim 1, characterised in that it contains, as active ingredient, N-[2-(3,3,3-trifluoropropyl)-phenylsulfonyl]-N'-(4-methoxy-6-methyl-1,3,5-triazin-2-yl)-urea.

17. A process for producing the compounds of the formula I, characterised in that

a) a phenylsulfonamide of the formula II

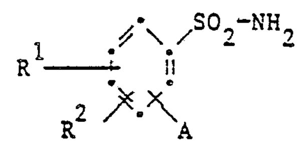

(II)

wherein A, $R^1$ and $R^2$ have the meanings defined under the formula I, is reacted in the presence of a base with an N-pyrimidinyl- or -triazinylcarbamate of the formula III

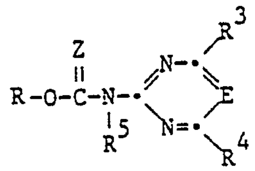

(III)

wherein E, $R^3$, $R^4$, $R^5$ and Z have the meanings defined under the formula I, and R is phenyl, alkyl or substituted phenyl, or

b) a phenylsulfonylisocyanate or -isothiocyanate of the formula IV

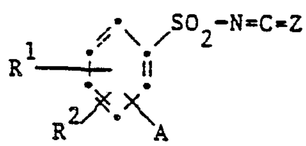

(IV)

wherein A, $R^1$, $R^2$ and Z have the meanings defined under the formula I, is reacted, optionally in the presence of a base, with an amine of the formula V

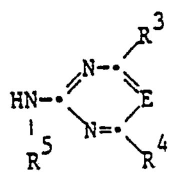

(V)

wherein E, $R^3$, $R^4$ and $R^5$ have the meanings defined under the formula I, or

c) an N-phenylsulfonylcarbamate of the formula VII

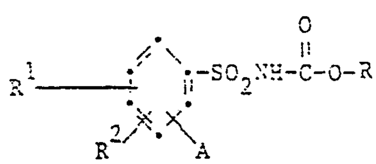

(VII)

wherein A, $R^1$ and $R^2$ have the meanings defined under the formula I, and R is phenyl, alkyl or substituted phenyl, is reacted with an amine of the formula V, and the products obtained are optionally converted into salts thereof by reacting a sulfonylurea of the formula I with an amine, with an alkali metal hydroxide or alkaline earth metal hydroxide or with a quaternary ammonium base.

18. The use of the N-phenylsulfonyl-N'-triazinyl- or -pyrimidinylureas of the formula I, according to claim 1, or of compositions containing them for controlling undesired plant growth.

19. The use of the N-phenylsulfonyl-N'-triazinyl- or -pyrimidinylureas of the formula I, according to claim 1, or of compositions containing them for regulating plant growth.

20. The use according to claim 18 for the selective, pre- or post-emergence controlling of weeds in crops of useful plants.

21. The use according to claim 20 in crops of sugar cane, cereals, maize and cotton.

22. The use according to claim 20 in soya-bean crops.
23. The use according to claim 20 in rice crops.
24. The use according to claim 19 in leguminous cover crops.

**Revendications** (pour les Etats contractants : BE, DE, FR, IT, NL, CH, LI, GB)

1. N-phénylsulfonyl-N'-pyrimidinyl- et -triazinyl-urées de formule I

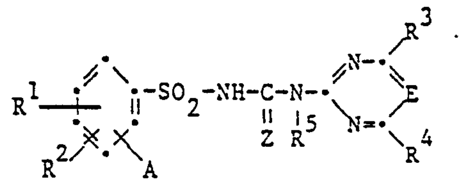

(I)

dans laquelle

A représente un groupe halogénoalkyle en C 1-C 6,

$R^1$ représente l'hydrogène, un halogène, un groupe nitro, cyano, alkyle en C 1-C 4, halogénoalkyle en C 1-C 4, alcoxy en C 1-C 4, alkylthio en C 1-C 4, alkylsulfinyle en C 1-C 4, alkylsulfonyle en C 1-C 4, $-CO-R^6$, $-NR^7R^8$, $-CO-NR^9R^{10}$ ou $-SO_2-NR^{11}R^{12}$,

$R^2$ représente l'hydrogène, un halogène, un groupe alkyle en C 1-C 4, alcoxy en C 1-C 4, alkylthio en C 1-C 4, alkylsulfinyle en C 1-C 4 ou alkylsulfonyle en C 1-C 4,

$R^3$ et $R^4$ représentent chacun, indépendamment l'un de l'autre, l'hydrogène, un halogène, un groupe alkyle en C 1-C 4, halogénoalkyle en C 1-C 4, halogénoalcoxy en C 2-C 4, alkylthio en C 1-C 4, halogénoalkylthio en C 1-C 4, alcoxyalkyle en C 2-C 4, méthoxy, éthoxy ou $-NR^{12}R^{13}$,

$R^5$ représente l'hydrogène, un groupe alkyle en C 1-C 4, alcoxy en C 1-C 4,

$R^6$ représente un groupe alcoxy en C 1-C 4, halogénoalcoxy en C 1-C 4, alkylthio en C 1-C 4, alcoxyalcoxy en C 2-C 6, l'hydrogène, un groupe alkyle en C 1-C 4 ou halogénoalkyle en C 1-C 4,

$R^7$, $R^8$, $R^9$, $R^{10}$, $R^{11}$, $R^{12}$ et $R^{13}$ représentent chacun, indépendamment les uns des autres, l'hydrogène ou un groupe alkyle en C 1-C 4,

E représente l'azote ou le pont méthine, et

Z représente l'oxygène ou le soufre,

et les sels de ces composés, sous réserve que A ne peut représenter un groupe trifluorométhyle, 2-fluoréthyle, 2-chloréthyle, 2-brométhyle, 2-chloro-2-fluoréthyle, 2,2-dichloréthyle, 2-bromo-2-chloréthyle, 2-bromo-2-fluoréthyle, 2,2-dibrométhyle, 2-fluoropropyle, 2-chloropropyle, 2-bromopropyle, ou un groupe $-CR^aR^bR^c$ dans lequel $R^a$ représente l'hydrogène, le chlore ou un groupe alkyle en C 1-C 4, $R^b$ l'hydrogène ou un groupe méthyle et $R^c$ le chlore ou le brome.

2. Composés selon la revendication 1, caractérisés en ce que $R^1$ représente l'hydrogène.

3. Composés selon la revendication 1, caractérisés en ce que $R^2$ représente l'hydrogène.

4. Composés selon la revendication 1, caractérisés en ce que Z représente l'oxygène.

5. Composés selon la revendication 1, caractérisés en ce que $R^5$ représente l'hydrogène.

6. Composés selon la revendication 1, caractérisés en ce que $R^3$ et $R^4$ ont ensemble au maximum 4 atomes de carbone.

7. Composés selon la revendication 1, caractérisés en ce que le groupe A contient au moins un atome de fluor.

8. Composés selon la revendication 1, caractérisés en ce que le groupe A occupe la position 2 du cycle phényle.

9. Composés selon la revendication 1, caractérisés en ce que $R^1$, $R^2$ et $R^5$ représentent l'hydrogène et Z l'oxygène.

10. Composés selon la revendication 1, caractérisés en ce que $R^3$ et $R^4$ ont ensemble au maximum 4 atomes de carbone, le groupe A est en position 2 du cycle phényle et contient au moins un atome de fluor.

11. Composés selon la revendication 1, caractérisés en ce que $R^1$, $R^2$ et $R^5$ représentent l'hydrogène et Z l'oxygène, $R^3$ et $R^4$ ont au maximum ensemble 4 atomes de carbone et le groupe A occupe la position 2 du cycle phényle et contient au moins un atome de fluor.

12. Composés selon la revendication 11, caractérisés en ce que A représente un groupe choisi parmi les groupes 3,3,3-trifluoropropyle, fluorométhyle, difluorométhyle, 3,3,4,4,5,5,5-heptafluoropentyle, 4,4,4-trifluoro-2-trifluorométhyle-butyle, chlorofluorométhyle, bromofluorométhyle, 3,3-difluorobutyle ou 3-fluoropropyle.

13. La N-[2-(3,3,3-trifluoropropyl)-phénylsulfonyl]-N'-(4,6-diméthoxy-pyrimidine-2-yl)-urée selon la revendication 1.

14. La N-[2-(3,3,3-trifluoropropyl)-phénylsulfonyl]-N'-(4,6-diméthoxy-1,3,5-triazine-2-yl)-urée selon la revendication 1.

15. La N-[2-(3,3-difluorobutyl)-phénylsulfonyl]-N'-(4-méthoxy-6-méthyl-1,3,5-triazine-2-yl)-urée selon la revendication 1.

16. La N-[2-(3,3,3-trifluoropropyl)-phénylsulfonyl]-N'-(4-méthoxy-6-méthyl-1,3,5-triazine-2-yl)-urée selon la revendication 1.

17. Phénylsulfonamides de formule II

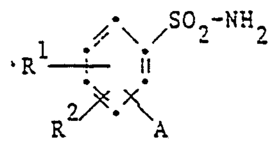

(II)

dans laquelle $R^1$, $R^2$ et A ont les significations indiquées en référence à la formule I dans la revendication 1.

18. Phénylsulfonylisocyanates et -isothiocyanates de formule IV

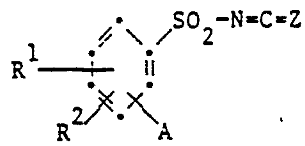

(IV)

dans laquelle $R^1$, $R^2$, A et Z ont les significations indiquées en référence à la formule I dans la revendication 1.

19. N-phénylsulfonylcarbamates de formule VII

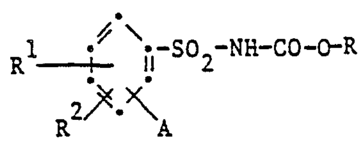

(VII)

dans laquelle $R^1$, $R^2$ et A ont les significations indiquées en référence à la formule I dans la revendication 1 et R représente un groupe phényle, alkyle ou phényle substitué.

20. Procédé de préparation des composés de formule I, caractérisé en ce que l'on fait réagir un phénylsulfonamide de formule II

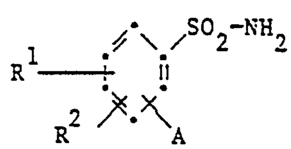

(II)

dans laquelle A, $R^1$ et $R^2$ ont les significations indiquées en référence à la formule I, en présence d'une base, avec un N-pyrimidinyl- ou -triazinyl-carbamate de formule III

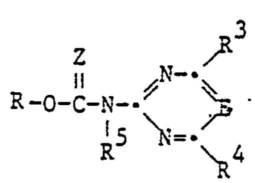

(III)

dans laquelle E, $R^3$, $R^4$, $R^5$ et Z ont les significations indiquées en référence à la formule I et R représente un groupe phényle, alkyle ou phényl substitué, et le cas échéant on convertit les composés obtenus en leurs sels.

21. Procédé de préparation des composés de formule I, caractérisé en ce que l'on fait réagir un phénylsulfonylisocyanate ou -isothiocyanate de formule IV

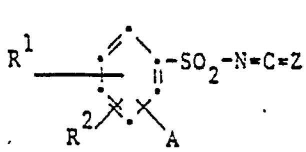

(IV)

dans laquelle A, $R^1$, $R^2$ et Z ont les significations indiquées en référence à la formule I, éventuellement en présence d'une base, avec une amine de formule V

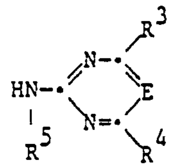

(V)

dans laquelle E, $R^3$, $R^4$ et $R^5$ ont les significations indiquées en référence à la formule I, et le cas échéant on convertit les composés obtenus en leurs sels.

22. Procédé de préparation des composés de formule I dans laquelle $R^5$ représente l'hydrogène, caractérisé en ce que l'on fait réagir un sulfonamide de formule II selon la revendication 20, éventuellement en présence d'une base, avec un isocyanate ou isothiocyanate de formule VI

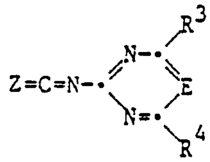

(VI)

dans laquelle E, $R^3$, $R^4$, $R^5$ et Z ont les significations indiquées en référence à la formule I, et le cas échéant, on convertit les composés obtenus en leurs sels.

23. Procédé de préparation des composés de formule I, caractérisé en ce que l'on fait réagir un N-phénylsulfonylcarbamate de formule VII

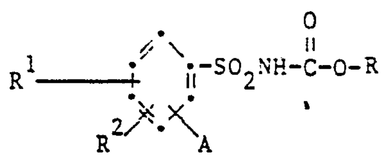

(VII)

dans laquelle A, $R^1$ et $R^2$ ont les significations indiquées en référence à la formule I et R représente un groupe phényle, alkyle ou phényle substitué, avec une amine de formule V selon la revendication 21, et le cas échéant, on convertit les composés obtenus en leurs sels.

24. Procédé de préparation des composés de formule I dans laquelle A représente un groupe halogénoalkyle en C 2-C 6, caractérisé en ce que l'on hydrogène ou l'on halogène jusqu'à saturation une sulfonylurée de formule VIII

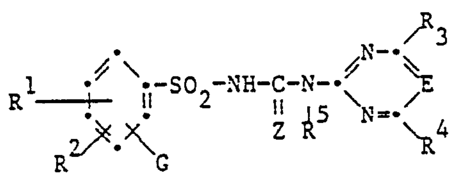

(VIII)

dans laquelle $R^1$, $R^2$, $R^3$, $R^4$, $R^5$, E et Z ont les significations indiquées en référence à la formule I et G représente un groupe halogénoalcényle insaturé en C 2-C 6, et le cas échéant, on convertit les composés obtenus en leurs sels.

25. Procédé de préparation des composés de formule I dans laquelle A représente un groupe halogénoalkyle en C 2-C 6, caractérisé en ce que l'on halogène jusqu'à saturation une sulfonylurée de formule IX

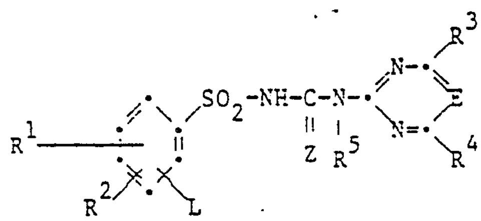

(IX)

dans laquelle $R^1$, $R^2$, $R^3$, $R^4$, $R^5$, E et Z ont les significations indiquées en référence à la formule I et L

représente un groupe alkyle insaturé en C 2-C 6, et le cas échéant on convertit les composés obtenus en leurs sels.

26. Procédé de préparation des sels d'addition de formule I selon l'une des revendications 20 à 25, caractérisé en ce que l'on fait réagir une sulfonylurée de formule I avec une amine, un hydroxyde de métal alcalin ou alcalino-terreux ou une base d'ammonium quaternaire.

27. Un produit herbicide et régulateur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I, selon la revendication 1.

28. Utilisation des N-phénylsulfonyl-N'-triazinyl- ou -pyrimidinyl-urées de formule I, selon la revendication 1, ou de produits en contenant dans la lutte contre les croissances de végétaux indésirables.

29. L'utilisation des N-phénylsulfonyl-N'-triazinyl- ou -pyrimidinyl-urées de formule I, selon la revendication 1, ou de produits en contenant pour la régulation de la croissance des végétaux.

30. L'utilisation des N-phénylsulfonyl-N'-triazinyl- ou -pyrimidinyl-urées de formule I ou de produits en contenant selon la revendication 28, pour la lutte sélective en pré-levée ou en post-levée contre les mauvaises herbes dans les cultures de végétaux utiles.

31. L'utilisation selon la revendication 30, dans les cultures de canne à sucre, de céréales, de maïs et de coton.

32. L'utilisation selon la revendication 30, dans les cultures de soja.

33. L'utilisation selon la revendication 30, dans les cultures de riz.

34. L'utilisation selon la revendication 29, sur les légumineuses de couvertures de sol.

35. Chlorures de phénylsulfonyle de formule

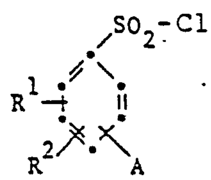

dans laquelle $R^1$, $R^2$ et A ont les significations indiquées en référence à la formule I dans la revendication 1.

**Revendications** (pour l'Etat contractant AT)

1. Un produit herbicide et régulateur de la croissance des végétaux, caractérisé en ce qu'il contient en tant que substance active, avec des véhicules et/ou d'autres additifs, au moins une N-phénylsulfonyl-N'-triazinyl- ou -pyrimidinyl-urée de formule I

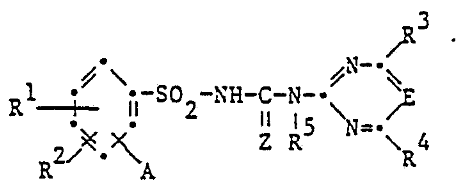

(I)

ou un sel d'un tel composé, dans lesquels

A représente un groupe halogénoalkyle en C 1-C 6,

$R^1$ représente l'hydrogène, un halogène, un groupe nitro, cyano, alkyle en C 1-C 4, halogénoalkyle en C 1-C 4, alcoxy en C 1-C 4, alkylthio en C 1-C 4, alkylsulfinyle en C 1-C 4, alkylsulfonyle en C 1-C 4, $-CO-R^6$, $-NR^7R^8$, $-CO-NR^9R^{10}$ ou $-SO_2-NR^{11}R^{12}$,

$R^2$ représente l'hydrogène, un halogène, un groupe alkyle en C 1-C 4, alcoxy en C 1-C 4, alkylthio en C 1-C 4, alkylsulfinyle en C 1-C 4 ou alkylsulfonyle en C 1-C 4,

$R^3$ et $R^4$ représentent chacun, indépendamment l'un de l'autre, l'hydrogène, un halogène, un groupe alkyle en C 1-C 4, halogénoalkyle en C 1-C 4, halogénoalcoxy en C 2-C 4, alkylthio en C 1-C 4, halogénoalkylthio en C 1-C 4, alcoxyalkyle en C 2-C 4, méthoxy, éthoxy ou $-NR^{12}R^{13}$,

$R^5$ représente l'hydrogène, un groupe alkyle en C 1-C 4 ou alcoxy en C 1-C 4,

$R^6$ représente un groupe alcoxy en C 1-C 4, halogénoalcoxy en C 1-C 4, alkylthio en C 1-C 4, alcoxyalcoxy en C 2-C 6, l'hydrogène, un groupe alkyle en C 1-C 4 ou halogénoalkyle en C 1-C 4,

$R^7$, $R^8$, $R^9$, $R^{10}$, $R^{11}$, $R^{12}$ et $R^{13}$ représentent chacun, indépendamment les uns des autres, l'hydrogène ou un groupe alkyle en C 1-C 4,

E représente l'azote ou le pont méthine, et

Z représente l'oxygène ou le soufre,

sous réserve que A ne peut représenter un groupe trifluorométhyle, 2-fluoréthyle, 2-chloréthyle, 2-brométhyle, 2-chloro-2-fluoréthyle, 2,2-dichloréthyle, 2-bromo-2-chloréthyle, 2-bromo-2-fluoréthyle, 2,2-dibrométhyle, 2-fluoropropyle, 2-chloropropyle, 2-bromopropyle ou un groupe $-CR^aR^bR^c$ dans lequel $R^a$ représente l'hydrogène, le chlore ou un groupe alkyle en C 1-C 4, $R^b$ l'hydrogène ou un groupe méthyle et $R^c$ le chlore ou le brome.

2.. Produit selon la revendication 1, caractérisé en ce que $R^1$ représente l'hydrogène.

3. Produit selon la revendication 1, caractérisé en ce que $R^2$ représente l'hydrogène.

4. Produit selon la revendication 1, caractérisé en ce que Z représente l'oxygène.

5. Produit selon la revendication 1, caractérisé en ce que $R^5$ représente l'hydrogène.

6. Produit selon la revendication 1, caractérisé en ce que $R^3$ et $R^4$ ont ensemble au maximum 4 atomes de carbone.

7. Produit selon la revendication 1, caractérisé en ce que le groupe A contient au moins un atome de fluor.

8. Produit selon la revendication 1, caractérisé en ce que le groupe A occupe la position 2 du cycle phényle.

9. Produit selon la revendication 1, caractérisé en ce que $R^1$, $R^2$ et $R^5$ représentent l'hydrogène et Z l'oxygène.

10. Produit selon la revendication 1, caractérisé en ce que $R^3$ et $R^4$ ont ensemble au maximum 4 atomes de carbone, le groupe A occupe la position 2 du cycle phényle et contient au moins un atome de fluor.

11. Produit selon la revendication 1, caractérisé en ce que $R^1$, $R^2$ et $R^5$ représentent l'hydrogène et Z l'oxygène, $R^3$ et $R^4$ ont ensemble au maximum 4 atomes de carbone et le groupe A occupe la position 2 du cycle phényle et contient au moins un atome de fluor.

12. Produit selon la revendication 11, caractérisé en ce que le groupe A est choisi parmi les groupes 3,3,3-trifluoropropyle, fluorométhyle, difluorométhyle, 3,3,4,4,5,5,5-heptafluoropentyle, 4,4,4-trifluoro-2-trifluorométhyl-butyle, chlorofluorométhyle, bromofluorométhyle, 3,3-difluorobutyle ou 3-fluoropropyle.

13. Produit selon la revendication 1, caractérisé en ce qu'il contient en tant que substance active la N-[2-(3,3,3-trifluoropropyl)-phénylsulfonyl]-N'-(4,6-diméthoxy-pyrimidine-2-yl)-urée.

14. Produit selon la revendication 1, caractérisé en ce qu'il contient en tant que substance active la N-[2-(3,3,3-trifluoropropyl)-phénylsulfonyl]-N'-(4,6-diméthoxy-1,3,5-triazine-2-yl)-urée.

15. Produit selon la revendication 1, caractérisé en ce qu'il contient en tant que substance active la N-[2-(3,3-difluorobutyl)-phénylsulfonyl]-N'-(4-méthoxy-6-méthyl-1,3,5-triazine-2-yl)-urée.

16. Produit selon la revendication 1, caractérisé en ce qu'il contient en tant que substance active la N-[2-(3,3,3-trifluoropropyl)-phénylsulfonyl]-N'-(4-méthoxy-6-méthyl-1,3,5-triazine-2-yl)-urée.

17. Procédé de préparation des composés de formule I, caractérisé en ce que :

a) on fait réagir un phénylsulfonamide de formule II ;

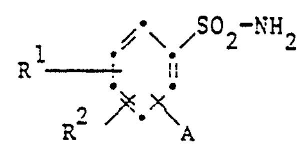

(II)

dans laquelle A, $R^1$ et $R^2$ ont les signifcations indiquées en référence à la formule I, en présence d'une base, avec un N-pyrimidinyl- ou triazinyl-carbamate de formule III

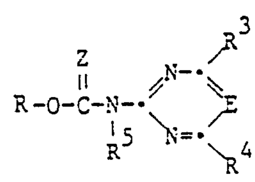

(III)

dans laquelle E, $R^3$, $R^4$, $R^5$ et Z ont les significations indiquées en référence à la formule I et R représente un groupe phényl, alkyle ou phényl substitué, ou bien

b) on fait réagir un phénylsulfonylisocyanate ou -isothiocyanate de formule IV

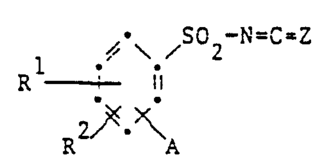

(IV)

dans laquelle A, $R^1$, $R^2$ et Z ont les significations indiquées en référence à la formule I, éventuellement en présence d'une base, avec une amine de formule V

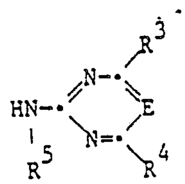

(V)

dans laquelle E, $R^3$, $R^4$ et $R^5$ ont les significations indiquées en référence à la formule I, ou bien

c) on fait réagir un N-phénylsulfonylcarbamate de formule VII

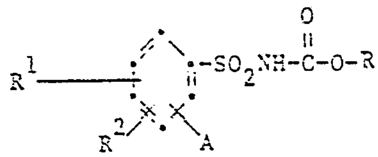

(VII)

dans laquelle A, $R^1$ et $R^2$ ont les significations indiquées en référence à la formule I et R représente un groupe phényle, alkyle ou phényle substitué, avec une amine de formule V, et le cas échéant, on convertit les composés obtenus en leurs sels en faisant réagir une sulfonylurée de formule I avec une amine, un hydroxyde de métal alcalin ou alcalino-terreux ou une base d'ammonium quaternaire.

18. L'utilisation des N-phénylsulfonyl-N'-triazinyl- ou pyrimidinyl-urées de formule I selon la revendication 1 ou de produits en contenant dans la lutte contre les croissances de végétaux indésirables.

19. L'utilisation des N-phénylsulfonyl-N'-triazinyl- ou -pyrimidinyl-urées de formule I selon revendication 1 ou de produits en contenant pour la régulation de la croissance des végétaux.

20. L'utilisation selon la revendication 18, pour la lutte sélective en prélevée ou en post-levée contre les mauvaises herbes dans les cultures de végétaux utiles.

21. L'utilisation selon la revendication 20, dans les cultures de canne à sucre, de céréales, de maïs et de coton.

22. L'utilisation selon la revendication 20, dans les cultures de soja.

23. L'utilisation selon la revendication 20, dans les cultures de riz.

24. L'utilisation selon la revendication 19, sur les légumineuses de couvertures de sol.